# EUROPEAN PATENT APPLICATION

(11) **EP 4 317 440 A1**
(43) Date of publication of application: **07.02.2024**
(21) Application number: 22778921.1
(22) Date of filing: 29.03.2022
(51) Int. Cl.: C12N 15/63, A61P 35/00, C12N 15/113, A61K 31/713

(54) **VIRAL VECTOR-BASED RNA DELIVERY SYSTEM AND USE THEREOF**

(30) Priority: 29.03.2021 CN 202110336982
(71) Applicant: Nanjing University, Nanjing, Jiangsu 210023 (CN)
(72) Inventor: ZHANG, Chenyu, Nanjing, Jiangsu 210023 (CN); CHEN, Xi, Nanjing, Jiangsu 210023 (CN); FU, Zheng, Nanjing, Jiangsu 210023 (CN); LI, Jing, Nanjing, Jiangsu 210023 (CN); ZHANG, Xiang, Nanjing, Jiangsu 210023 (CN); ZHOU, Xinyan, Nanjing, Jiangsu 210023 (CN); ZHANG, Li, Nanjing, Jiangsu 210023 (CN); YU, Mengchao, Nanjing, Jiangsu 210023 (CN); GUO, Hongyuan, Nanjing, Jiangsu 210023 (CN)
(74) Representative: Cabinet Laurent & Charras
(86) International application number: PCT/CN2022/083601
(87) International publication number: WO 2022/206739

(57) **Abstract**

Provided are a viral vector-based RNA delivery system and a use thereof. The RNA delivery system comprises a viral vector. The viral vector carries an RNA fragment needing to be delivered, and can be enriched in organ tissues of a host. In the host organ tissues, the viral vector can endogenously and spontaneously form a composite structure containing the RNA fragment. The composite structure can enter and bind to target tissues, and feed the RNA fragment into the target tissues. The viral vector has a targeting tag. The composite structure is an exosome. The viral vector RNA delivery system is safe and reliable, and has good druggability and high universality.

## Description

### FIELD

The present application relates to the technical field of biomedicine, and specifically relates to a viral vector-based RNA delivery system and a use thereof.

### BACKGROUND

RNA interference (RNAi) therapy has been considered a promising strategy to treat human diseases since its invention. However, it has faced numerous issues during clinical practice, and the progress of the therapy's development has fallen short of expectations.

It is generally believed that RNA cannot remain stable outside the cell for a long time, because RNA will be degraded into fragments due to the abundance of RNase in the extracellular environment. It is therefore crucial to find a method that can stabilize RNA outside the cell and enable it to enter targeted tissues, thereby enhancing the effect of RNAi therapy.

There are currently several patents concerning siRNA, mainly focusing on the following aspects: 1. Designing siRNA for medical application. 2. Chemically modifying siRNA in order to enhance its stability *in vivo* and increase the yield. 3. Refining the design of various artificial carriers, including lipid nanoparticles, cationic polymers and viruses, to improve the efficiency of siRNA delivery *in vivo.* Among them, there are a number of patents regarding the third aspect. The primary cause for this is that researchers have realized the absence of suitable siRNA delivery systems for safely, accurately and efficiently deliver siRNA to target tissues. This challenge has become the fundamental limitation of RNAi therapy.

Biological viruses are small, simple-structured, non-cellular organisms that contain only one type of nucleic acid (DNA or RNA) and must be parasitic in living cells and multiply by replication. Viral vectors can bring genetic material into cells. The principle is to use the molecular mechanism of viruses to transfer their genomes into other cells for infection. This can occur in intact living organisms (in vivo) or in cell cultures (in vitro). It is mainly used in basic research, gene therapy or vaccines. However, there are currently few studies on using viruses as carriers to deliver RNA, especially siRNA, by special self-assembly mechanisms.

Chinese patent CN108624590A discloses an siRNA capable of inhibiting the expression of DDR2 gene. Chinese patent CN108624591A discloses an siRNA capable of silencing ARPC4 gene, and the siRNA is modified with α-phosphorus-selenium. Chinese patent CN108546702A discloses an siRNA targeting a long non-coding RNA, DDX11-AS1. Chinese patent CN106177990A discloses an siRNA precursor that can be used for the treatment of various tumors. These patents devise specific siRNAs and target certain diseases caused by genetic mutations.

Chinese patent CN108250267A discloses a polypeptide and a polypeptide-siRNA induced co-assembly, where the polypeptide acts as a carrier of siRNA. Chinese patent CN108117585A discloses a polypeptide to target and to introduce siRNA for promoting apoptosis of breast cancer cells, also utilizing the polypeptide as a carrier of siRNA. Chinese patent CN108096583A discloses a nanoparticle carrier, which can be loaded with siRNA that has curative effect on breast cancer while containing chemotherapeutic drugs. These patents are all inventions on siRNA carriers, whose technical solutions share the common feature of pre-assembling the carrier and siRNA *in vitro* before introducing them to the host. In fact, this is characteristic for most of the currently designed delivery techniques. However, these delivery systems pose a common issue that such artificially synthesized exogenous delivery system is prone to be cleared by the host's circulatory system, cause immunogenic reactions, and even potentially be toxic to certain cell types and tissues.

The research team of the present invention found that endogenous cells can selectively encapsulate miRNAs into exosomes. Exosomes can deliver miRNA to receptor cells, and the secreted miRNA can effectively block the expression of target genes at a relatively low concentration. Exosomes are biocompatible with the host immune system and possess the inherent ability to protect and transport miRNA across biological barriers in vivo, thereby having the potential to overcome problems associated with siRNA delivery. For example, Chinese patent CN110699382A discloses a method for preparing exosomes delivering siRNA, which involves techniques of isolating exosomes from plasma and encapsulating siRNA into exosomes by electroporation.

However, such techniques for isolating or preparing exosomes in vitro often necessitate obtaining a large amount of exosomes through cell culture, together with the step of siRNA encapsulation, which makes the clinical cost of large-scale application of the product too high for patients to afford. More importantly, the intricate production/purification process of exosomes makes it almost impossible to comply with GMP standards.

So far, medicinal products containing exosomes as active ingredients have not received approval from CFDA. The main challenge lies in ensuring the consistency of exosome products, which results in these products failing to obtain drug production licenses. If this issue can be resolved, it would significantly advance RNAi therapy.

Therefore, the development of a safe, precise and efficient siRNA delivery system is a crucial part to improve the efficacy of RNAi therapy and advance it further.

### SUMMARY

In view of this, the embodiments of the present application provide a viral vector-based RNA delivery system and a use thereof to solve the technical deficiencies existing in the existing technology.

The present application provides a viral vector-based RNA delivery system, comprising a viral vector carrying an RNA fragment to be delivered, wherein the viral vector is capable of enriching and endogenously spontaneously forming a complex comprising the RNA fragment in a host organ or tissue, and the complex is capable of entering and binding to a target tissue, and delivering the RNA fragment into the target tissue. After the RNA fragment is delivered to the target tissue, it can inhibit the expression of its matching gene, thereby inhibiting the disease development in the target tissue.

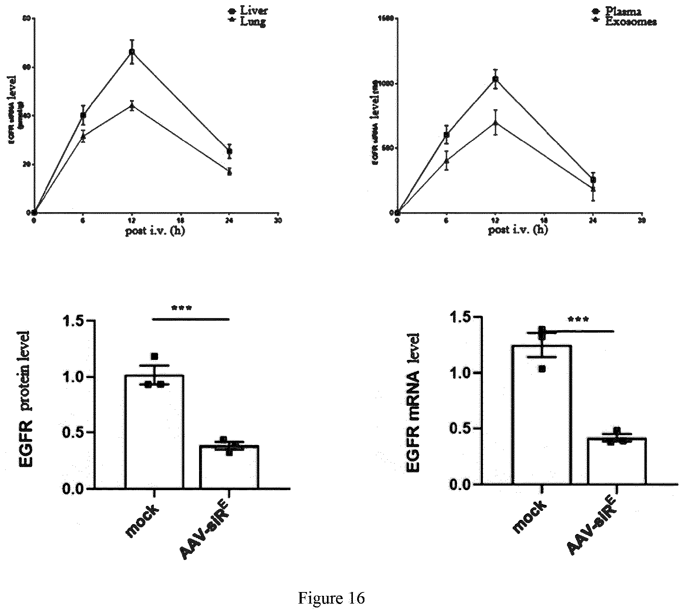

Figures 16-17 show that the RNA delivery system constructed with adeno-associated viral vectors or lentiviral vectors has enrichment effect and therapeutic effect in vivo.

Optionally, the viral vector is an adenovirus-associated virus.

Optionally, the adenovirus-associated virus is adenovirus-associated virus type 5, adenovirus-associated virus type 8 or adenovirus-associated virus type 9.

Optionally, the RNA fragment comprises one, two or more RNA sequences with medical significance, and the RNA sequence is siRNA, shRNA or miRNA with medical significance.

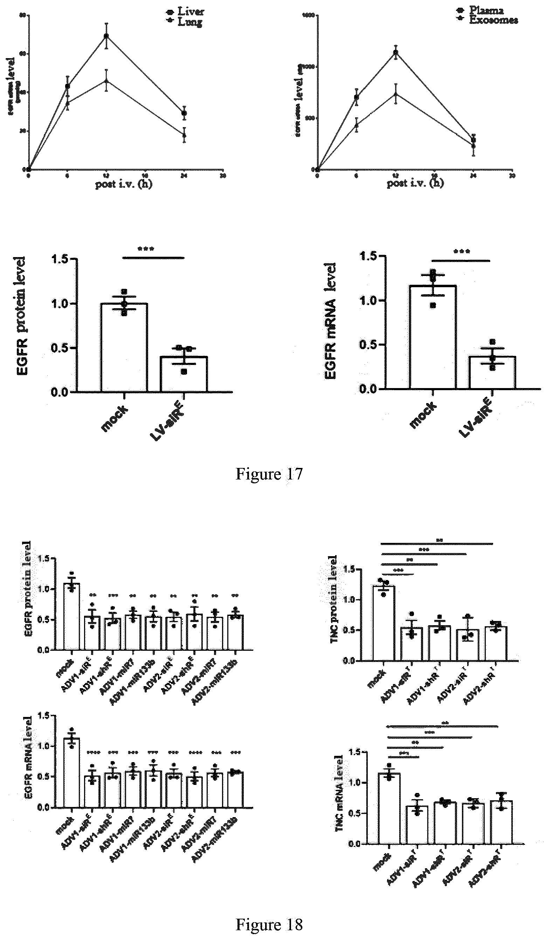

Figures 18-19 show that the RNA delivery system constructed with two adenoviral vectors and the RNA fragment composed of six RNA sequences or any two or three of the six RNA sequences has enrichment effect and therapeutic effect in vivo.

Optionally, the viral vector comprises a promoter and a targeting tag, wherein the targeting tag is capable of forming a targeting structure of the complex in the host organ or tissue, the targeting tag is located on the surface of the complex, and the complex seeks for and binds to the target tissue through the targeting structure, and delivers the RNA fragment into the target tissue.

Optionally, the viral vector comprises a circuit selected from the group consisting of promoter-RNA fragment, promoter-targeting tag, and promoter-RNA fragment-targeting tag, or a combination thereof; and the viral vector comprises at least an RNA fragment and a targeting tag, wherein the RNA fragment and the targeting tag are located in the same circuit or in different circuits.

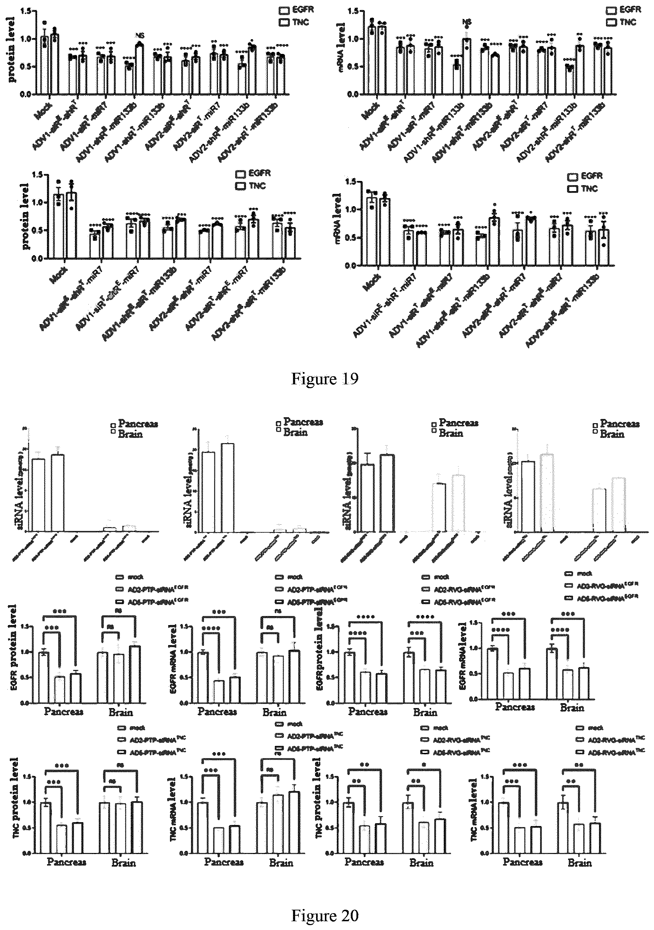

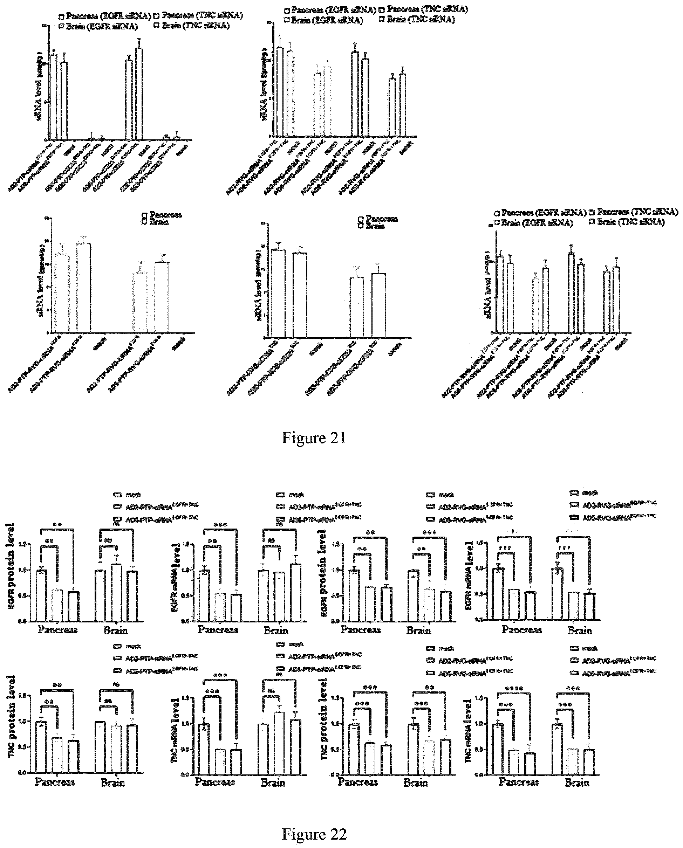

Figures 20-23 show that the RNA delivery system constructed with two RNA fragments and two targeting tags alone or in any combination has enrichment effect and therapeutic effect in vivo.

Optionally, the viral vector further comprises a flanking sequence, a compensation sequence and a loop sequence that facilitate the correct folding and expression of the circuit, and the flanking sequence comprises 5' flanking sequence and 3' flanking sequence;
the viral vector comprises a circuit selected from the group consisting of 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence, or a combination thereof.

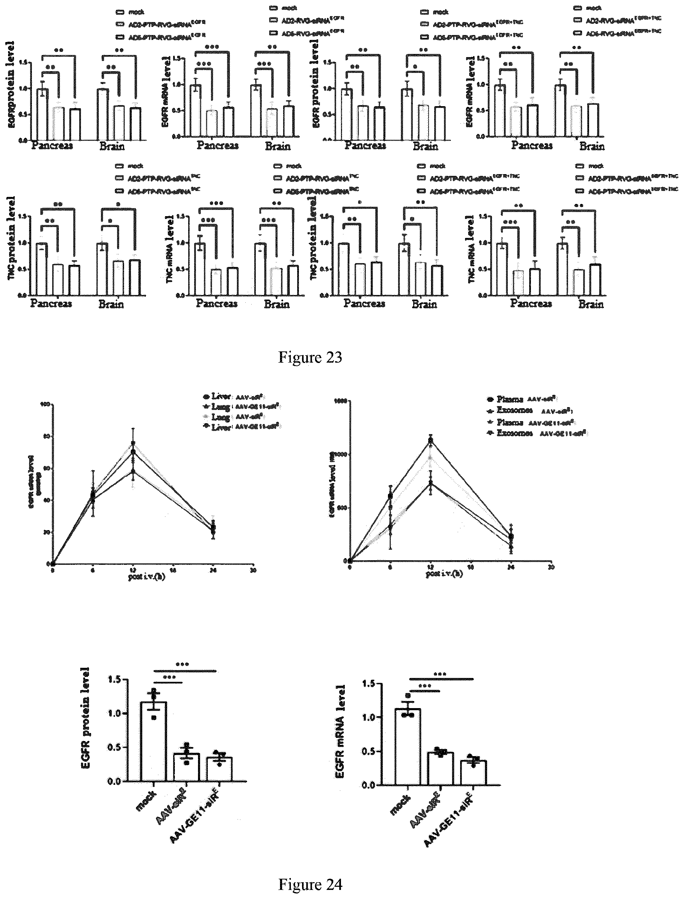

Figure 24 shows that the delivery system constructed with 5' promoter-5' flanking sequence-RNA sequence-loop sequence-compensation sequence-3' flanking sequence or 5' promoter-targeting tag-5' flanking sequence-RNA sequence-loop sequence-compensation sequence-3' flanking sequence has enrichment effect and therapeutic effect in vivo.

Optionally, the 5' flanking sequence has a sequence that is identical to or more than 80% homologous with ggatcctggaggcttgctgaaggctgtatgctgaattc;
the loop sequence has a sequence that is identical to or more than 80% homologous with gttttggccactgactgac;
the 3' flanking sequence has a sequence that is identical to or more than 80% homologous with accggtcaggacacaaggcctgttactagcactcacatggaacaaatggcccagatctggccgcactcgag;
the compensation sequence has a reverse complementary sequence to the RNA fragment in which any 1 to 5 bases have been deleted, so that the compensation sequence is not expressed.

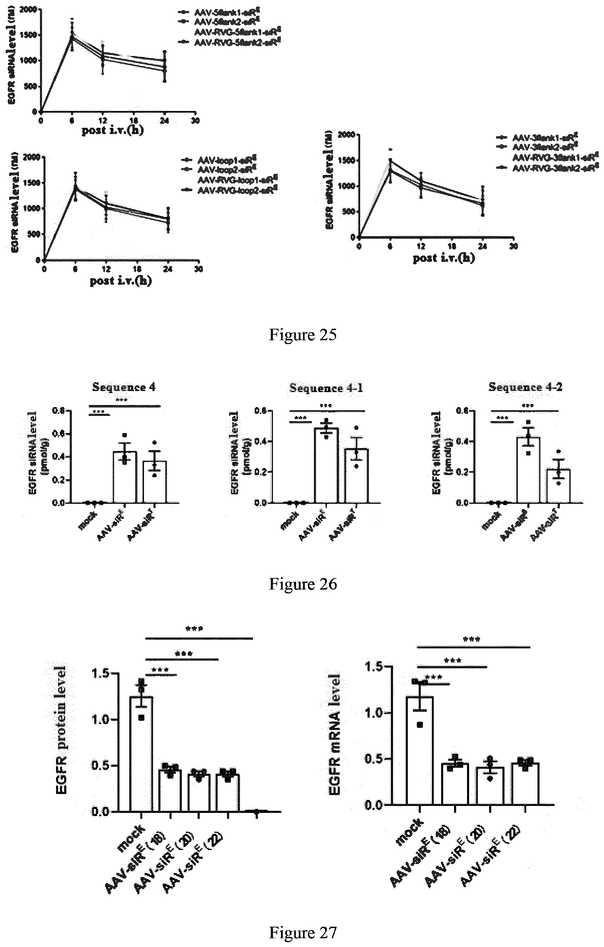

Figure 25 shows that the delivery system constructed with 5' flanking sequence/loop sequence/3' flanking sequence or its homologous sequence has enrichment effect and therapeutic effect in vivo.

Preferably, the compensation sequence has a reverse complementary sequence to the RNA fragment in which any 1 to 3 bases have been deleted.

More preferably, the compensation sequence has a reverse complementary sequence to the RNA fragment in which any 1 to 3 consecutively arranged bases have been deleted.

Most preferably, the compensation sequence has a reverse complementary sequence to the RNA fragment in which bases at positions 9 and 10 have been deleted.

Optionally, in the case where the viral vector comprises two or more of the circuits, adjacent circuits are linked via a sequence composed of sequences 1-3 (sequence 1-sequence 2-sequence 3);
wherein, sequence 1 is CAGATC, sequence 2 is a sequence of 5-80 bases, and sequence 3 is TGGATC.

Optionally, in the case where the viral vector comprises two or more of the gene circuits, adjacent gene circuits are linked via sequence 4 or a sequence with more than 80% homology to sequence 4;
wherein, sequence 4 is CAGATCTGGCCGCACTCGAGGTAGTGAGTCGACCAGTGGATC.

Figure 26 shows that the viral vector delivery system constructed with sequence 4 or its homologous sequence has enrichment effect and therapeutic effect in vivo.

Optionally, the organ or tissue is liver, and the complex is an exosome.

Optionally, the targeting tag is a targeting peptide or targeting protein with targeting function.

Optionally, the targeting peptide is selected from the group consisting of RVG targeting peptide, GE11 targeting peptide, PTP targeting peptide, TCP-1 targeting peptide, and MSP targeting peptide;
the targeting protein is selected from the group consisting of RVG-LAMP2B fusion protein, GE11-LAMP2B fusion protein, PTP-LAMP2B fusion protein, TCP-1-LAMP2B fusion protein, and MSP-LAMP2B fusion protein.

Optionally, the RNA sequence is 15-25 nucleotides in length. For example, the RNA sequence may be 16, 17, 18, 19, 20, 21, 22, 23, 24, or 25 nucleotides in length. Preferably, the RNA sequence is 18-22 nucleotides in length.

Figure 67 shows the gene circuits constructed with RNA sequences of different lengths all have enrichment effect and therapeutic effect in vivo.

Optionally, the RNA sequence has a sequence that is identical to, more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof. It should be noted that the RNA sequence in "a nucleic acid molecule encoding a sequence selected from" the following herein also includes RNA sequences with more than 80% homology to the above-mentioned RNA sequence.

Wherein, the siRNAs of the above-mentioned genes are RNA sequences that have the function of inhibiting the expression of the gene. Numerous RNA sequences with this function exist and cannot all be listed here, and the following sequences with better effects are provided as examples.

The siRNA of the EGFR gene has a sequence that is identical to or more than 80% homologous with UGUUGCUUCUCUUAAUUCCU, AAAUGAUCUUCAAAAGUGCCC, UCUUUAAGAAGGAAAGAUCAU, AAUAUUCGUAGCAUUUAUGGA, UAAAAAUCCUCACAUAUACUU, or other sequences that inhibit EGFR gene expression.

The siRNA of the KRAS gene has a sequence that is identical to or more than 80% homologous with UGAUUUAGUAUUAUUUAUGGC, AAUUUGUUCUCUAUAAUGGUG, UAAUUUGUUCUCUAUAAUGGU, UUAUGUUUUCGAAUUUCUCGA, UGUAUUUACAUAAUUACACAC, or other sequences that inhibit KRAS gene expression.

The siRNA of the VEGFR gene has a sequence that is identical to or more than 80% homologous with AUUUGAAGAGUUGUAUUAGCC, UAAUAGACUGGUAACUUUCAU, ACAACUAUGUACAUAAUAGAC, UUUAAGACAAGCUUUUCUCCA, AACAAAAGGUUUUUCAUGGAC, or other sequences that inhibit VEGFR gene expression.

The siRNA of the mTOR gene has a sequence that is identical to or more than 80% homologous with AGAUAGUUGGCAAAUCUGCCA, ACUAUUUCAUCCAUAUAAGGU, AAAAUGUUGUCAAAGAAGGGU, AAAAAUGUUGUCAAAGAAGGG, UGAUUUCUUCCAUUUCUUCUC, or other sequences that inhibit mTOR gene expression.

The siRNA of the TNF-α gene has a sequence that is identical to or more than 80% homologous with AAAACAUAAUCAAAAGAAGGC, UAAAAAACAUAAUCAAAAGAA, AAUAAUAAAUAAUCACAAGUG, UUUUCACGGAAAACAUGUCUG, AAACAUAAUCAAAAGAAGGCA, or other sequences that inhibit TNF-α gene expression.

The siRNA of the integrin-α gene has a sequence that is identical to or more than 80% homologous with AUAAUCAUCUCCAUUAAUGUC, AAACAAUUCCUUUUUUAUCUU, AUUAAAACAGGAAACUUUGAG, AUAAUGAAGGAUAUACAACAG, UUCUUUAUUCAUAAAAGUCUC, or other sequences that inhibit integrin-α gene expression.

The siRNA of the B7 gene has a sequence that is identical to or more than 80% homologous with UUUUCUUUGGGUAAUCUUCAG, AGAAAAAUUCCACUUUUUCUU, AUUUCAAAGUCAGAUAUACUA, ACAAAAAUUCCAUUUACUGAG, AUUAUUGAGUUAAGUAUUCCU, or other sequences that inhibit B7 gene expression.

The siRNA of the TGF-β1 gene has a sequence that is identical to or more than 80% homologous with ACGGAAAUAACCUAGAUGGGC, UGAACUUGUCAUAGAUUUCGU, UUGAAGAACAUAUAUAUGCUG, UCUAACUACAGUAGUGUUCCC, UCUCAGACUCUGGGGCCUCAG, or other sequences that inhibit TGF-β1gene expression.

The siRNA of the H2-K gene has a sequence that is identical to or more than 80% homologous with AAAAACAAAUCAAUCAAACAA, UCAAAAAAACAAAUCAAUCAA, UAUGAGAAGACAUUGUCUGUC, AACAAUCAAGGUUACAUUCAA, ACAAAACCUCUAAGCAUUCUC, or other sequences that inhibit H2-K gene expression.

The siRNA of the H2-D gene has a sequence that is identical to or more than 80% homologous with AAUCUCGGAGAGACAUUUCAG, AAUGUUGUGUAAAGAGAACUG, AACAUCAGACAAUGUUGUGUA, UGUUAACAAUCAAGGUCACUU, AACAAAAAAACCUCUAAGCAU, or other sequences that inhibit H2-D gene expression.

The siRNA of the H2-L gene has a sequence that is identical to or more than 80% homologous with GAUCCGCUCCCAAUACUCCGG, AUCUGCGUGAUCCGCUCCCAA, UCGGAGAGACAUUUCAGAGCU, UCUCGGAGAGACAUUUCAGAG, AAUCUCGGAGAGACAUUUCAG, or other sequences that inhibit H2-L gene expression.

The siRNA of the HLA gene has a sequence that is identical to or more than 80% homologous with AUCUGGAUGGUGUGAGAACCG, UGUCACUGCUUGCAGCCUGAG, UCACAAAGGGAAGGGCAGGAA, UUGCAGAAACAAAGUCAGGGU, ACACGAACACAGACACAUGCA, or other sequences that inhibit HLA gene expression.

The siRNA of the GDF15 gene has a sequence that is identical to or more than 80% homologous with UAUAAAUACAGCUGUUUGGGC, AGACUUAUAUAAAUACAGCUG, AAUUAAUAAUAAAUAACAGAC, AUCUGAGAGCCAUUCACCGUC, UGCAACUCCAGCUGGGGCCGU, or other sequences that inhibit GDF15 gene expression.

The siRNA of the TNC gene has a sequence that is identical to or more than 80% homologous with AUGAAAUGUAAAAAAAGGGA, AAUCAUAUCCUUAAAAUGGAA, UAAUCAUAUCCUUAAAAUGGA, UGAAAAAUCCUUAGUUUUCAU, AGAAGUAAAAAACUAUUGCGA, or other sequences that inhibit TNC gene expression.

The siRNA of the PTP1B gene has a sequence that is identical to or more than 80% homologous with UGAUAUAGUCAUUAUCUUCUU, UCCAUUUUUAUCAAACUAGCG, AUUGUUUAAAUAAAUAUGGAG, AAUUUUAAUACAUUAUUGGUU, UUUAUUAUUGUACUUUUUGAU, or other sequences that inhibit PTP1B gene expression.

The siRNA of the mHTT gene has a sequence that is identical to or more than 80% homologous with UAUGUUUUCACAUAUUGUCAG, AUUUAGUAGCCAACUAUAGAA, AUGUUUUUCAAUAAAUGUGCC, UAUGAAUAGCAUUCUUAUCUG, UAUUUGUUCCUCUUAAUACAA, or other sequences that inhibit mHTT gene expression.

The siRNA of the Lrrk2 gene has a sequence that is identical to or more than 80% homologous with AUUAACAUGAAAAUAUCACUU, UUAACAAUAUCAUAUAAUCUU, AUCUUUAAAAUUUGUUAACGC, UUGAUUUAAGAAAAUAGUCUC, UUUGAUAACAGUAUUUUUCUG, or other sequences that inhibit Lrrk2 gene expression.

The siRNA of the α-synuclein gene has a sequence that is identical to or more than 80% homologous with AUAUAUUAACAAAUUUCACAA, AAGUAUUAUAUAUAUUAACAA, AUAACUUUAUAUUUUUGUCCU, UAACUAAAAAAUUAUUUCGAG, UCGAAUAUUAUUUAUUGUCAG, or other sequences that inhibit α-synuclein gene expression.

The antisense strand of miRNA-21 is 5'-TCAACATCAGTCTGATAAGCTA-3'.

It should be noted that the above-mentioned "more than 80% homologous with" a sequence may refer to a homology of 85%, 88%, 90%, 95%, 98%, etc.

Optionally, the RNA fragment includes the RNA sequence and a modified RNA sequence obtained by ribose modification to the RNA sequence. That is to say, the RNA fragment may consist of at least one RNA sequence, at least one modified RNA sequence, or the RNA sequence and the modified RNA sequence.

In the present invention, the isolated nucleic acid also includes variants and derivatives thereof. Those skilled in the art can use common methods to modify the nucleic acid. Such modification includes (but not limited to) methylation modification, hydrocarbon modification, glycosylation modification (such as 2-methoxy-glycosyl modification, hydroxyl-glycosyl modification, saccharide ring modification), nucleic acid modification, peptide fragment modification, lipid modification, halogen modification, and nucleic acid modification (such as "TT" modification). In one embodiment of the present invention, the modification is an internucleotide linkage, for example selected from the group consisting of phosphorothioate, 2'-O methoxyethyl (MOE), 2'-fluoro, alkyl phosphonate, phosphorodithioate, alkyl phosphonothioate, phosphoramidate, carbamate, carbonate, phosphotriester, acetamidate, carboxymethyl ester, and combinations thereof. In one embodiment of the present invention, the modification is a modification of nucleotides, for example, selected from the group consisting of peptide nucleic acid (PNA), locked nucleic acid (LNA), arabinose nucleic acid (FANA), analogs and derivatives thereof, and combinations thereof. Preferably, the modification is 2' fluoropyrimidine modification. 2' Fluoropyrimidine modification is to replace the 2'-OH of the pyrimidine nucleotide of RNA with 2'-F. 2'-F modification can make RNA difficult to be recognized by RNase in vivo, thereby increasing the stability of RNA fragment during transportation in vivo.

Optionally, the delivery system is a delivery system for use in a mammal including human.

The present application also provides use of the viral vector-based RNA delivery system as described in any paragraph above in the manufacture of a medicament for treating a disease.

Optionally, the medicament is administered by oral administration, inhalation, subcutaneous injection, intramuscular injection, or intravenous injection, preferably by intravenous injection.

Optionally, the disease is a cancer, pulmonary fibrosis, colitis, obesity, cardiovascular disease caused by obesity, type 2 diabetes, Huntington's disease, Parkinson's disease, myasthenia gravis, Alzheimer's disease, or graft-versus-host disease.

Optionally, the medicament comprises the above-mentioned viral vector. Specifically, the viral vector herein refers to a viral vector carrying an RNA fragment, or carrying an RNA fragment and a targeting tag. It can enter the host and is capable of enriching in the liver and self-assembling into a complex, an exosome. The complex can deliver the RNA fragment to the target tissue, allowing the RNA fragment to be expressed in the target tissues, thereby inhibiting the expression of its matching gene and achieving the purpose of treating diseases.

The dosage form of the medicament may be tablets, capsules, powders, granules, pills, suppositories, ointments, solutions, suspensions, lotions, gels, pastes, etc.

**The technical effects of this application include:**
The viral vector-based RNA delivery system provided in the present application uses a virus as a carrier, and as a mature injection, the virus has safety and reliability that have been fully verified, and has a very good druggability. The RNA sequence that exerts the final effect is encapsulated and transported by endogenous exosomes, with no immune responses, and there is no need to verify the safety of the exosomes. The delivery system can deliver all kinds of small molecule RNAs and has strong versatility. Moreover, the preparation of viruses is much cheaper than the preparation of exosomes, proteins, polypeptides and the like, with good economy. The viral vector-based RNA delivery system provided in the present application can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

The use of the viral vector-based RNA delivery system provided in this application in the medicament offers a platform of drug delivery that facilitates the establishment of further foundations for RNA drug research and development. This significantly advances the development and utilization of RNA drugs.

### BRIEF DESCRIPTION OF DRAWINGS

Figure 1 shows the comparison of the treatment of colitis and RNA expression levels in mice provided by an embodiment of the present application.
Figure 2 shows the comparison of mouse cytokine concentration and colon HE staining provided by an embodiment of the present application.
Figure 3 shows the comparison of the treatment of colitis in mice provided by an embodiment of the present application.
Figure 4 shows the comparison of the disease activity index and various siRNA levels in mice provided by an embodiment of the present application.
Figure 5 shows the comparison of various siRNA and mRNA levels in mice provided by an embodiment of the present application.
Figure 6 shows the comparison of HE staining of mouse colon provided by an embodiment of the present application.
Figure 7 shows the treatment of lung cancer in mice based on KRAS siRNA provided by an embodiment of the present application.
Figure 8 shows the treatment of lung cancer in mice based on EGFR siRNA provided by an embodiment of the present application.
Figure 9 shows the comparison of multiple enzyme contents in mice provided by an embodiment of the present application.
Figure 10 shows the comparison of hydroxyproline content and mRNA levels in mice provided by an embodiment of the present application.
Figure 11 shows the comparison of mouse survival and tumor evaluation provided by an embodiment of the present application.
Figure 12 shows the comparison of the treatment of obesity in mice provided by an embodiment of the present application.
Figure 13 shows the comparison of various obesity indicators in mice provided by an embodiment of the present application.
Figure 14 shows the comparison of the treatment of Huntington's disease in mice provided by an embodiment of the present application.
Figure 15 shows the construction and characterization of the circuit provided by an embodiment of the present application.
Figure 16 shows the enrichment effect of the adeno-associated viral vector and expression levels of EGFR gene in the liver, lung, plasma and exosomes provided by an embodiment of the present application; where A shows the enrichment effect of the adeno-associated viral vector carrying RNA sequence in the liver and lung, B shows the enrichment effect of the adeno-associated viral vector carrying RNA sequence in the plasma and exosomes, and C and D show the protein and mRNA expression levels of EGFR.
Figure 17 shows the enrichment effect of the lentiviral vector and expression levels of EGFR gene in the liver, lung, plasma and exosomes provided by an embodiment of the present application; where A shows the enrichment effect of the lentiviral vector carrying RNA sequence in the liver and lung, B shows the enrichment effect of the lentiviral vector carrying RNA sequence in the plasma and exosomes, and C and D show the protein and mRNA expression levels of EGFR.
Figure 18 shows the detection results of corresponding protein and mRNA levels after intravenous injection of gene circuits constructed with two different adenoviral vectors and containing six different RNA sequences provided by an embodiment of the present application; where A shows the expression level of EGFR protein, B shows the expression level of TNC protein, C shows the expression level of EGFR mRNA, and D shows the expression level of TNC mRNA.
Figure 19 shows the protein levels after intravenous injection of delivery systems constructed with two adenoviral vectors and the RNA fragment composed of any two or three of six RNA sequences provided by an embodiment of the present application; where A shows the protein levels of EGFR and TNC with the delivery system constructed with two adenoviral vectors and the RNA fragment composed of any two of six RNA sequences, B shows the mRNA levels of EGFR and TNC with the delivery system constructed with two adenoviral vectors and the RNA fragment composed of any two of six RNA sequences, C shows the protein levels of EGFR and TNC with the delivery system constructed with two adenoviral vectors and the RNA fragment composed of any three of six RNA sequences, and D shows the mRNA levels of EGFR and TNC with the delivery system constructed with two adenoviral vectors and the RNA fragment composed of any four of six RNA sequences.
Figure 20 shows the enrichment effect of EGFR siRNA and TNC siRNA and expression levels of EGFR and TNC in the pancreas and brain after intravenous injection of the delivery system in which a single RNA fragment and a single targeting tag are combined and constructed into a viral vector provided by an embodiment of the present application; where A-D show the enrichment effect of AD2/AD5+PTP/RVG+siR^{EGFR}/siR^{TNC}, E-H show the protein and mRNA levels of EGFR with AD2/AD5+PTP/RVG+siR^{EGFR}, and I-L show the protein and mRNA levels of TNC with AD2/AD5+PTP/RVG+siR^{TNC}.
Figure 21 shows the enrichment effect of EGFR siRNA and TNC siRNA in the pancreas and brain after intravenous injection of the delivery system in which two RNA fragments and two targeting tags are arbitrarily combined and constructed into a viral vector provided by an embodiment of the present application; where A-B show the enrichment effect of AD2/AD5+PTP/RVG+siR^{EGFR+TNC}, C-D show the enrichment effect of AD2/AD5+(PTP-RVG)+siR^{EGFR}, and E shows the enrichment effect of AD2/AD5+(PTP-RVG)+siR^{EGFR+TNC}.
Figure 22 shows the expression levels of EGFR and TNC in the pancreas and brain after intravenous injection of the delivery system in which two RNA fragments and two targeting tags are arbitrarily combined and constructed into a viral vector provided by an embodiment of the present application; where A-D show the protein and mRNA levels of EGFR with AD2/AD5+PTP/RVG+siR^{EGFR+TNC}, and E-H show the protein and mRNA levels of TNC.
Figure 23 shows the expression levels of EGFR and TNC in the pancreas and brain after intravenous injection of the delivery system in which two RNA fragments and two targeting tags are arbitrarily combined and constructed into a viral vector provided by another example of the present application; where A-B show the protein and mRNA levels of EGFR with AD2/AD5+(PTP-RVG)+siR^{EGFR}, C-D show the protein and mRNA levels of EGFR with AD2/AD5+(PTP-RVG)+siR^{EGFR+TNC}, and E-F show the protein and mRNA levels of TNC with AD2/AD5+(PTP-RVG)+siR^{TNC}, and G-H show the protein and mRNA levels of TNC with AD2/AD5+(PTP-RVG)+siR^{EGFR+TNC}.
Figure 24 shows the enrichment effect and protein and mRNA levels of EGFR of siRNA in the liver, lung, plasma, and exosomes after intravenous injection of the delivery systems constructed with two circuits provided by an embodiment of the present application; where A shows the enrichment effect of the delivery system AAV-siR^{E} and AAV-GE11-siR^{E} in the liver and lung, B shows the enrichment effect of the delivery system AAV-siR^{E} and AAV-GE11-siR^{E} in the plasma and exosomes, C shows the protein level of EGFR with the delivery system AAV-siR^{E} and AAV-GE11-siR^{E}, and D shows the mRNA level of EGFR with the delivery system AAV-siR^{E} and AAV-GE11-siR^{E}.
Figure 25 shows the enrichment effect of the adenoviral vector containing two specific sequences with more than 80% homology to the 5' flanking sequence/loop sequence/3' flanking sequence in the lung provided by an embodiment of the present application, where A shows the enrichment effect of different 5' flanking sequences, B shows the enrichment effect of different loop sequences, and C shows the enrichment effect of different 3' flanking sequences.
Figure 26 shows the enrichment effect in the lung after 9 h of intravenous injection of the delivery system in which sequence 4 or two sequences 4-1 and 4-2 with more than 80% homology to sequence 4 was constructed into an AAV vector provided by an embodiment of the present application; where A shows siRNA levels of AAV-siR^{E} and AAV-siR^{T} linked by sequence 4, B shows siRNA levels of AAV-siR^{E} and AAV-siR^{T} linked by sequence 4-1, and C shows siRNA levels of AAV-siR^{E} and AAV-siR^{T} linked by sequence 4-2.
Figure 27 shows the expression levels of EGFR after intravenous injection of gene circuits containing sequences of different lengths provided by an embodiment of the present application; where A shows the protein level of EGFR, and B shows the mRNA level of EGFR.

### DETAILED DESCRIPTION

Specific embodiments of the present application will be described below in conjunction with the accompanying drawings.

First, the technical terms, experimental methods, etc. involved in the present invention are explained.

Hematoxylin-eosin staining, referred to as HE staining, is one of the most basic and widely used technical methods in the teaching and scientific research of histology and pathology.

Hematoxylin stain is alkaline and can stain the basophilic structures of tissues (such as ribosomes, nuclei and ribonucleic acid in the cytoplasm, etc.) into blue-purple, and eosin is an acidic dye that can stain eosinophilic structures of tissues (such as intracellular and intercellular proteins, Lewy bodies, Mallory bodies, and most of the cytoplasm) into pink, making the morphology of the entire cell tissue clearly visible.

The specific steps of HE staining include: sample tissue fixation and sectioning; tissue sample deparaffinizing; tissue sample hydration; hematoxylin staining of tissue sections, differentiation and anti-blue; eosin staining of tissue sections and dehydration; air-drying and sealing tissue sample sections; and finally, observation and taking pictures under a microscope.

Western Blot transfers proteins to a membrane and then uses antibodies for detection. For known expressed proteins, the corresponding antibodies can be used as primary antibodies for detection. For the expression products of new genes, the antibodies against the fusion part can be used for detection.

In Western Blot, polyacrylamide gel electrophoresis is employed, where the detected object is protein, the "probe" is an antibody, and the "color development" uses a labeled secondary antibody. The protein sample separated by PAGE is transferred to a solid-phase carrier (such as nitrocellulose film), where it is absorbed through non-covalent bonds without changing the type or biological activity of the polypeptide separated by electrophoresis. The protein or peptide on the solid-phase carrier acts as an antigen and reacts immunologically with the corresponding antibody. Then, it reacts with the second antibody labeled with either enzyme or isotope. The protein component expressed by the specific target gene is separated through electrophoresis and detected through substrate color development or autoradiography. The process primarily comprises of extracting proteins, quantifying proteins, preparing gels, running electrophoresis, transferring membranes, performing immunolabeling, and developing.

Immunohistochemistry (also known as immunocytochemistry), based on the antigen-antibody reaction, i.e., the principle of specific binding of antigen and antibody, is the process of identifying antigens (polypeptides or proteins) in tissue cells, determining their localization, and studying them qualitatively and relative quantitatively by chemical reaction of the chromogenic agent (fluorescein, enzyme, metal ion, or isotope) of the labeled antibody to develop color.

The main steps of immunohistochemistry include soaking sections, airing overnight, deparaffinizing in xylene followed by alcohol of gradient concentrations (100%, 95%, 90%, 80%, 75%, 70%, 50%, 3 min each time), using double distilled water, adding dropwise 3% hydrogen peroxide solution to remove catalase, washing with water, repairing antigen, adding dropwise 5% BSA, blocking for 1h, diluting primary antibody, washing with PBS buffer, incubating with secondary antibody, washing with PBS buffer, developing color with chromogenic solution, washing with water, staining with hematoxylin, dehydrating with ethanol of gradient concentrations, and sealing with neutral gum.

The detection of siRNA, protein and mRNA levels involved in the present invention is all performed by injecting an RNA delivery system into mice to establish an in vitro mouse stem cell model. qRT-PCR is used to detect the expression levels of mRNA and siRNA in cells and tissues. The absolute quantification of siRNA is determined by drawing a standard curve with a standard product. The expression level of each siRNA or mRNA relative to the internal reference can be represented by 2-ΔCT, where ΔCT=Csample-Cinternal reference. The internal reference gene used is U6 snRNA (in tissue) or miR-16 (in serum, exosomes) molecules when amplifying siRNA, and is GAPDH or 18s RNA when amplifying mRNA. Western blotting is used to detect the expression level of proteins in cells and tissues, and ImageJ software is used for protein quantitative analysis.

In the figures provided by the present invention, "*" means P < 0.05, "**" means P < 0.01, and "***" means P < 0.005.

In the present invention, unless otherwise specified, the scientific and technical terms used herein have the meanings commonly understood by those skilled in the art. Moreover, the reagents, materials and procedures used herein are all reagents, materials and procedures widely used in the corresponding fields.

### Example 1

In this example, a viral vector-based RNA delivery system is provided, which comprises a viral vector carrying an RNA fragment to be delivered, wherein the viral vector is capable of enriching and endogenously spontaneously forming a complex comprising the RNA fragment in a host organ or tissue, and the complex is capable of entering and binding to a target tissue, and delivering the RNA fragment into the target tissue.

Figures 16-17 show that the RNA delivery system constructed with adeno-associated viral vectors and lentiviral vectors has enrichment effect and therapeutic effect in vivo. Figure 16 shows the enrichment effect of the adeno-associated viral vector and expression levels of EGFR gene in the liver, lung, plasma and exosomes. Figure 17 shows the enrichment effect of the lentiviral vector and expression levels of EGFR gene in the liver, lung, plasma and exosomes.

In this example, the viral vector further comprises a promoter and a targeting tag. The viral vector comprises a circuit selected from the group consisting of promoter-RNA sequence, promoter-targeting tag, and promoter-RNA sequence-targeting tag, or a combination thereof; and the viral vector comprises at least an RNA fragment and a targeting tag, wherein the RNA fragment and the targeting tag are located in the same circuit or in different circuits. In other words, the viral vector may only comprise promoter-RNA sequence-targeting tag, or a combination of promoter-RNA sequence and promoter-targeting tag, or a combination of promoter-targeting tag and promoter-RNA sequence-targeting tag.

In Figures 20-23, RNA fragment 1 is siR^{EGFR}, RNA fragment 2 is siR^{TNC}, targeting tag 1 is PTP, and targeting tag 2 is RVG. After the two RNA fragments and the two targeting tags were used alone or in any combination, constructed into a viral vector, and injected into mice through the tail vein, the enrichment effect of EGFR siRNA and TNC siRNA and expression levels of EGFR and TNC in the pancreas, brain, plasma and exosomes were detected.

Further, the viral vector can also comprise a flanking sequence, a compensation sequence and a loop sequence that facilitate the correct folding and expression of the circuit, and the flanking sequence comprises 5' flanking sequence and 3' flanking sequence; the viral vector comprises a circuit selected from the group consisting of 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence, or a combination thereof.

Wherein, the 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment with any 1-5 bases deleted. In the case where the RNA fragment contains only one RNA sequence, the compensation sequence may be the reverse complementary sequence of the RNA sequence with any 1-5 bases deleted.

Preferably, the compensation sequence is the reverse complementary sequence of the RNA fragment with any 1-3 bases deleted. In the case where the RNA fragment contains only one RNA sequence, the compensation sequence may be the reverse complementary sequence of the RNA sequence with any 1-3 bases deleted.

More preferably, the compensation sequence is the reverse complementary sequence of the RNA fragment with any 1-3 contiguous bases deleted. In the case where the RNA fragment contains only one RNA sequence, the compensation sequence may be the reverse complementary sequence of the RNA sequence with any 1-3 contiguous bases deleted.

Most preferably, the compensation sequence is the reverse complementary sequence of the RNA fragment with the base at position 9 and/or 10 deleted. In the case where the RNA fragment contains only one RNA sequence, the compensation sequence may be a reverse complementary sequence of the RNA sequence with the base at position 9 and/or 10 deleted. Deleting the base at position 9 and/or 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

In Figure 24, the delivery vector corresponding to 5' promoter-5' flanking sequence-RNA sequence-loop sequence-compensation sequence-3' flanking sequence is AAV-siR^{E}, and the delivery vector corresponding to 5'-promoter-targeting tag-5' flanking sequence-RNA sequence-loop sequence-compensation sequence-3' flanking sequence is AAV-GE11-siR^{E}. Figure 24 shows the enrichment effect and protein and mRNA levels of EGFR of siRNA in the liver, lung, plasma, and exosomes after intravenous injection.

Figure 25 shows a set of data on the enrichment effect of the adenoviral vector in the lung. The sequences in the adenoviral vectors are: two specific sequences two specific sequences with more than 80% homology to the 5' flanking sequence/loop sequence/3' flanking sequence.

The above sequences are specifically shown in Table 1 below.

**Table 1**

| **Name** | **Sequence** |
|---|---|
| 5' flanking sequence-1 | CTGGAGGCTTGCTGAAGGCTGTATGCTGAATTCG |
| 5' flanking sequence-2 | CTGGAGGCTTGCTCGGAGGCTGTATGCTGGCTTCG |
| loop-1 | GTTTTGGCCACTGACTGAC |
| loop-2 | GTGGCGGCCACTGACTGAC |
| 3' flanking sequence-1 | CACCGGTCAGGACACAAGGCCTGTTACTAGCACTCACATGGAA CAAATGGCC |
| 3' flanking sequence-2 | CAGAGGTCAGGACACAAGGCCTACGACTAGCACTCACATGTCT CAAATGGCC |

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in Table 2 below.

**Table 2**

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

Figure 26 shows the siRNA levels of EGFR in the lung after 9 h of intravenous injection of the delivery system in which sequence 4 or two sequences 4-1 and 4-2 with more than 80% homology to sequence 4 was constructed into an AAV vector.

The sequences are specifically shown in Table 3 below.

**Table 3**

| **Name** | **Sequence** |
|---|---|
| Sequence 4 | CAGATCTGGCCGCACTCGAGGTAGTGAGTCGACCAGTGGATC |
| Sequence 4-1 | CAGATCTGCTCTAACTCGATTTAGTGAGTCGACCAGTGGATC |
| Sequence 4-2 | CAGATCTGGTTTCACTCATTCTAGTGAGTCGACCAGTGGATC |

The RNA fragment may comprise one, two or more RNA sequences with medical significance. The RNA sequence can be expressed in the target receptor, and the compensation sequence cannot be expressed in the target receptor. The RNA sequence may be an siRNA sequence, shRNA sequence or miRNA sequence, preferably an siRNA sequence.

The length of an RNA sequence is 15-25 nucleotides (nt), preferably 18-22 nt, such as 18 nt, 19 nt, 20 nt, 21 nt, or 22 nt. The range of the sequence length is not chosen arbitrarily but was determined after repeated trials. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

Figure 27 shows the expression levels of EGFR after intravenous injection of gene circuits constructed with RNA sequences of different lengths, where the plasmids with RNA sequence lengths of 18, 20, and 22 correspond to AAV-siR^{E} (18), AAV-siR^{E} (20), AAV-siR^{E} (22), respectively.

RNA sequences of different lengths are shown in Table 4 below.

**Table 4**

| **Name** | **Sequence** |
|---|---|
| siRE(18) | ACCTATTCCGTTACACACT |
| siRE(20) | ATACCTATTCCGTTACACAC |
| siRE(22) | ATACCTATTCCGTTACACACTT |

The RNA sequence has a sequence that is identical to, more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof.

The siRNAs and the antisense strands of miRNAs of the above-mentioned genes can inhibit the expression or mutation of the genes and miRNAs, thereby achieving the effect of inhibiting diseases. These diseases include, but are not limited to: cancer, pulmonary fibrosis, colitis, obesity, cardiovascular disease caused by obesity, type 2 diabetes, Huntington's disease, Parkinson's disease, myasthenia gravis, Alzheimer's disease, graft-versus-host disease and related diseases. The related diseases herein refer to the associated diseases, complications or sequelae that appear during the formation or development of any one or several of the above diseases, or other diseases that are related to the above diseases to a certain extent. Among them, cancers include but are not limited to: gastric cancer, kidney cancer, lung cancer, liver cancer, brain cancer, blood cancer, bowel cancer, skin cancer, lymphatic cancer, breast cancer, bladder cancer, esophageal cancer, head and neck squamous cell carcinoma, hemangioma, glioblastoma, or melanoma.

The number of RNA sequences in an RNA fragment is 1, 2 or more. For example, if glioma needs to be treated, siRNA of EGFR gene and siRNA of TNC gene can both be used in the same viral vector; and if enteritis needs to be treated, siRNA of TNF-α gene, siRNA of integrin-α gene and siRNA of B7 gene can all be used.

Taking the combined use of "siRNA1" and "siRNA2" on the same viral vector as an example, the functional structural region of the viral vector can be expressed as (promoter-siRNA 1)-linker-(promoter-siRNA 2)-linker-(promoter-targeting tag), or (promoter-targeting tag-siRNA1)-linker-(promoter-targeting tag-siRNA2), or (promoter-siRNA1)-linker-(promoter-targeting tag-siRNA 2), etc.

More specifically, the functional structural region of the viral vector can be expressed as (5'-promoter-5' flanking sequence-siRNA 1-loop sequence-compensation sequence-3' flanking sequence)-linker-(5'-promoter-5'flanking sequence-siRNA 2-loop sequence-compensation sequence-3' flanking sequence)-linker-(5'-promoter-targeting tag), or (5'-promoter-targeting tag-5' flanking sequence-siRNA 1-loop sequence-compensation sequence-3' flanking sequence)-linker-(5'-promoter-targeting tag-5' flanking sequence-siRNA 2-loop sequence-compensating sequence-3' flanking sequence), or (5'-promoter-5' flanking sequence-siRNA 1-loop sequence-compensation sequence-3' flanking sequence)-linker-(5'-promoter-targeting tag-5' flanking sequence-siRNA 2-loop sequence-compensation sequence-3' flanking sequence), or (5'-promoter-targeting tag-5' flanking sequence-siRNA 1- siRNA 2- loop sequence-compensation sequence-3' flanking sequence), etc. Other situations can be deduced in this way and will not be repeated here. The above linker can be "sequence 1-sequence 2-sequence 3" or "sequence 4", and a bracket indicates a complete circuit.

Preferably, the above-mentioned RNA can also be obtained by ribose modification of the RNA sequence (siRNA, shRNA or miRNA), preferably 2' fluoropyrimidine modification. 2' Fluoropyrimidine modification is to replace the 2'-OH of the pyrimidine nucleotide of siRNA, shRNA or miRNA with 2'-F. 2'-F modification can make siRNA, shRNA or miRNA difficult to be recognized by RNase in the human body, thereby increasing the stability of RNA during transportation in vivo.

Preferably, the above-mentioned RNA can also be obtained by ribose modification of the RNA sequence (siRNA, shRNA or miRNA), preferably 2' fluoropyrimidine modification. 2' Fluoropyrimidine modification is to replace the 2'-OH of the pyrimidine nucleotide of siRNA, shRNA or miRNA with 2'-F. 2'-F modification can make siRNA, shRNA or miRNA difficult to be recognized by RNase in the human body, thereby increasing the stability of RNA during transportation in vivo.

The host organ or tissue described in this example is preferably the liver. The liver is the most active cell opening and closing tissue among all mammalian tissues and organs.

After specifically selecting to infect the liver, the viral vector will release the RNA fragment (siRNA, shRNA or miRNA). The liver tissue can spontaneously encapsulate the RNA fragment into exosomes, and these exosomes become RNA delivery mechanisms (a complex structure containing the desired RNA to be delivered and having a targeting structure).

In order to enable the exosomes to have the ability of "precise guidance", a targeting tag can be designed in the virus injected into the body, and the targeting tag will also be assembled into the exosomes by the liver tissue, especially when selecting certain targeting tags, they can be inserted into the surface of exosomes to become a targeting structure that can guide exosomes. This can greatly improve the accuracy of the RNA delivery mechanism of the present invention and greatly improve the efficiency of potential drug delivery.

The selection of targeting tags is a process that requires creative work. On the one hand, it is necessary to select available targeting tags according to the target tissue, and on the other hand, it is also necessary to ensure that the targeting tag can stably appear on the surface of exosomes to achieve targeting function. Targeting tags that have been screened include targeting peptides, targeting proteins, and antibodies. Wherein, targeting peptides include but are not limited to RVG targeting peptide (with the nucleotide sequence as shown in SEQ ID No: 1), GE11 targeting peptide (with the nucleotide sequence as shown in SEQ ID No: 2), PTP targeting peptide (with the nucleotide sequence as shown in SEQ ID No: 3), TCP-1 targeting peptide (with the nucleotide sequence as shown in SEQ ID No: 4), or MSP targeting peptide (with the nucleotide sequence as shown in SEQ ID No: 5); targeting proteins include but are not limited to RVG-LAMP2B fusion protein (with the nucleotide sequence as shown in SEQ ID No: 6), GE11-LAMP2B fusion protein (with the nucleotide sequence as shown in SEQ ID No: 7), PTP-LAMP2B fusion protein (with the nucleotide sequence as shown in SEQ ID No: 8), TCP-1-LAMP2B fusion protein (with the nucleotide sequence as shown in SEQ ID No: 9), or MSP-LAMP2B fusion protein (with the nucleotide sequence as shown in SEQ ID No: 10).

Among them, RVG targeting peptide and RVG-LAMP2B fusion protein can precisely target brain tissue; GE11 targeting peptide and GE11-LAMP2B fusion protein can precisely target organs or tissues with high expression of EGFR, such as lung cancer tissue with EGFR mutation; PTP targeting peptide and PTP-LAMP2B fusion protein can precisely target the pancreas, especially the plectin-1 protein specifically expressed in human and mouse pancreatic cancer tissues; TCP-1 targeting peptide and TCP-1-LAMP2B fusion protein can precisely target the colon; and MSP targeting peptide and MSP-LAMP2B fusion protein can precisely target muscle tissue.

In practical applications, the targeting tag can be flexibly matched with various RNA fragments, and different targeting tags can play different roles with different RNA fragments. For example, RVG targeting peptide and RVG-LAMP2B fusion protein can be combined with siRNA of EGFR gene, siRNA of TNC gene or a combination of the two to treat glioblastoma, be combined with siRNA of PTP1B gene to treat obesity, be combined with siRNA of mHTT gene to treat Huntington's disease, or be combined with siRNA of LRRK2 gene to treat Parkinson's disease; GE11 targeting peptide and GE11-LAMP2B fusion protein can be combined with siRNA of EGFR gene to treat lung cancer and other diseases induced by high expression or mutation of EGFR gene; TCP-1 targeting peptide or TCP-1-LAMP2B fusion protein can be combined with siRNA of TNF-α gene, siRNA of integrin-α gene, siRNA of B7 gene or any combination of the above three to treat colitis or colon cancer.

The selection and structural design of viral vectors is one of the core contents of the technology disclosed in the present invention. After research, the viral vector is preferably adenovirus-associated virus (AAV), and more preferably adenovirus-associated virus type 5 (AAV-5), Adenovirus-associated virus type 8 (AAV-8) or adenovirus-associated virus type 9 (AAV-9).

In addition, in order to achieve the purpose of precise delivery, we experimented with a variety of viral vector loading schemes and came up with another optimized scheme: the viral vector can also be composed of a variety of viruses with different structures, one of which contains a promoter and targeting tag, other viruses contain promoters and RNA fragments. That is, the targeting tag and RNA fragment are loaded into different viral vectors, and the two viral vectors are injected into the body. The targeting effect is no worse than the targeting effect produced by loading the same targeting tag and RNA fragment into one viral vector.

More preferably, when two different viral vectors are injected into the host body, the viral vector containing the RNA sequence can be injected first, and then the viral vector containing the targeting tag can be injected 1-2 hours later, so that a better target effect can be achieved.

The delivery systems described above can be used in mammals including humans.

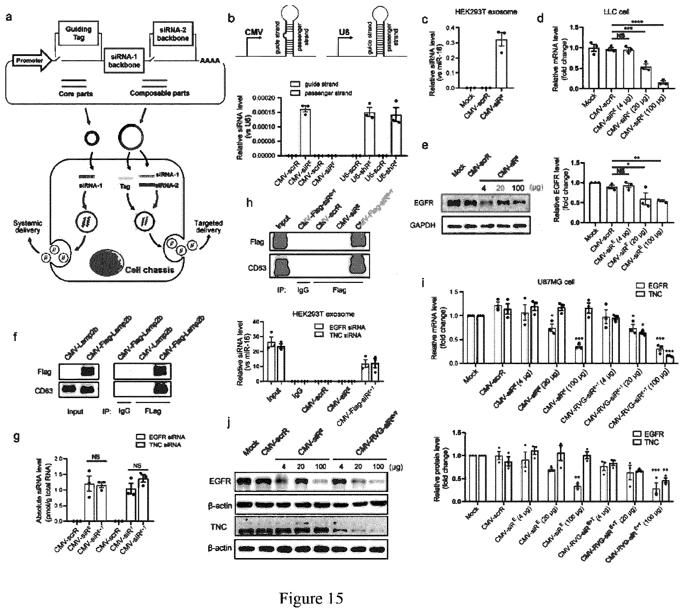

In order to verify the feasibility of the delivery system, we conducted the following experiments:
First, referring to Figure 15a, we rationally designed a basic gene circuit architecture that allows the free combination of different functional modules. The core circuit, consisting of a promoter part and an siRNA-expressing part, was designed to produce and organize siRNAs as the payload for exosomes. Other composable parts (plug-ins) could then be integrated into the framework of the core circuit to achieve plug-and-play functionality. As an example, two types of composable parts were incorporated to optimize the siRNA effects: one modifying the membrane-anchored proteins of exosomes to enable tissue selectivity; the other enabling the co-expression of a second siRNA for the simultaneous inhibition of two molecular targets.

For the core gene circuit construct, we designed a scheme that encodes an optimized siRNA expression backbone part under the control of a promoter part to maximize guide strand expression while minimizing undesired passenger strand expression, see Figure 15a. Epidermal growth factor receptor (EGFR), one of the most potent oncogenes that is frequently mutated and highly expressed in a variety of human cancers (e.g., lung cancer and glioblastoma), was selected as the siRNA target. Human embryonic kidney 293T cells (HEK293T) and mouse hepatoma cells (Hepa 1-6) were chosen as the cell chassis for in vitro assembly of siRNA. To optimize the siRNA production efficiency, we compared two classic design strategies: one using the CMV promoter to express an miRNA precursor (pre-miRNA) and substituting the miRNA sequence with siRNA, and the other using the U6 promoter to express a short hairpin RNA (shRNA). We first examined a series of pre-miRNA structures and selected pre-miR-155 as the optimal backbone for the production of siRNA. Then, the siRNA production efficiency of a CMV-directed pre-miRNA encoding an EGFR siRNA (CMV-siR^{E}) and a U6-directed EGFR shRNA (U6-shR^{E}) was compared, see Figure 15b. Both strategies had similar efficiencies in driving the transcription of the guide strand of EGFR siRNA; however, compared with the shRNA approach, the pre-miRNA approach produced less or no passenger strand (the passenger strand is seemingly degraded during the biogenesis of mature guide strand), see Figure 15b. Therefore, the CMV-driven pre-miRNA design was chosen to avoid off-target effects.

Next, we examined whether the core circuit directs the loading of siRNA into exosomes. HEK293T cells were transfected with the control scrambled circuit (CMV-scrR) or CMV-siRE circuit, and the exosomes in cell culture medium were characterized. Nanoparticle tracking analysis (NTA) revealed that similar amounts of exosomes were secreted in each group with similar size and distribution, peaking at 128-131 nm. Transmission electron microscopy (TEM) confirmed that the purified exosomes exhibited a typical round-shaped vesicular morphology and had correct size. Moreover, enrichment of specific exosome markers (CD63, TSG101 and CD9) was only detected in purified exosomes but not in cell culture medium. These results demonstrate that transfection with circuits did not affect the size, structure or number of exosomes generated by HEK293T cells. Finally, a significant amount of EGFR siRNA was detected in exosomes derived from HEK293T and Hepa 1-6 cells transfected with the CMV-siR^{E} circuit, see Figure 15c. When these exosomes were incubated with mouse Lewis lung carcinoma (LLC) cells, a dose-dependent knockdown of EGFR expression was achieved, see Figure 15d and Figure 15e, suggesting that the exosomal siRNAs were biologically functional.

For the design of the targeting tag of the circuit, a sequence encoding a targeting tag fused to the N-terminus of Lamp2b (a canonical exosome membrane protein) was inserted downstream of the CMV promoter, see Figure 15a). This tag was anchored to the exosome surface via Lamp2b, thus guiding the delivery of the complex exosome to the desired tissue. Specifically, a central nervous system-targeting RVG peptide was chosen as the tag to direct exosomes to the brain (RVG has been shown to facilitate transcytosis of exosomes across the blood-brain barrier and into neuronal cells). The efficiency of the promoters in initiating the expression of the RVG-Lamp2b fusion protein was first assessed. Experiments have shown that the CMV promoter has a certain effect in generating RVG-Lamp2b mRNA and marker protein eGFP in HEK293T cells, whereas the U6 promoter was not effective, confirming the advantage of the CMV promoter for linking each part of the circuit. The correct display of the targeting tag on the exosome surface was then verified using immunoprecipitation. A Flag epitope tag was used to replace RVG because of the lack of an anti-RVG antibody. After transfection of HEK293T and Hepa 1-6 cells with a CMV-directed Flag-Lamp2b circuit, intact exosomes were successfully immunoprecipitated with anti-Flag beads, see Figure 15f, demonstrating the precise localization of the targeting tag. For the design of additional siRNA-expressing parts, tenascin-C (TNC), a critical oncogene associated with many cancers, especially glioblastoma, was selected as the second siRNA target. TNC siRNA was also embedded in the pre-miR-155 backbone and inserted downstream of the EGFR siRNA, see Figure 15. Regardless of the individual (CMV-siR^{E} or CMV-siR^{T}) or tandem (CMV-siR^{E}) transcription, equal amounts of EGFR and TNC siRNA were detected, see Figure 15g. Overall, these results show that both the siRNA and targeting tag can play their respective roles in the circuit.

Next, we examined whether the circuit enables the self-assembly of siRNAs into exosomes in vitro. HEK293T cells were transfected with a CMV-directed circuit encoding both EGFR and TNC siRNAs along with an RVG tag (CMV-RVG-siR^{E}). The results showed that exosomes derived from the cell culture medium displayed typical morphology and size distribution, indicating that modification with the composable-core circuit did not alter the physical properties of exosomes. Moreover, a complete circuit encoding both EGFR and TNC siRNAs and a targeting tag (CMV-Flag-siR^{E}) was constructed. Exosomes generated from the transfected HEK293T and hep 1-6 cells were successfully immunoprecipitated, and both EGFR and TNC siRNAs were abundantly present in the immunoprecipitated exosomes, see Figure 15h.

Furthermore, AGO2 is widely expressed in organisms and is a core component of the RNA-induced silencing complex. It has endoribonuclease activity and can inhibit the expression of target genes by promoting the maturation of siRNA and regulating its biosynthesis and function.

Since siRNA processing is dependent on Argonaute 2 (AGO2) and the proper loading of siRNA into AGO2 is expected to enhance the on-target effects of siRNA, another immunoprecipitation experiment was performed to assess the association of AGO2 with siRNAs in exosomes. Experiments demonstrated that EGFR and TNC siRNAs were readily detected in the exosomes precipitated with anti-AGO2 antibody, suggesting that our design guarantees the loading of siRNA into the RNA-induced silencing complex (RISC) and facilitates the efficient transport of AGO2-bound siRNA into exosomes. Finally, to investigate whether the in vitro assembled siRNAs are functional, exosomes derived from HEK293T cells transfected with the CMV-RVG-siR^{E+T} circuit were incubated with the U87MG glioblastoma cells. A dose-dependent downregulation of EGFR and TNC expression in U87MG cells was achieved, see Figure 15i and Figure 15j. Moreover, the RVG tag on exosome surface did not affect the silencing efficacy of EGFR and TNC siRNAs on their targets. These results establish the circuit as an organic combination of multiple composable parts that direct the self-assembly and release of siRNAs in vitro.

Therefore, the viral vector-based RNA delivery system provided in this example uses a virus as a carrier, and as a mature injection, the virus has safety and reliability that have been fully verified, and has a very good druggability. The RNA sequence that exerts the final effect is encapsulated and transported by endogenous exosomes, with no immune responses, and there is no need to verify the safety of the exosomes. The delivery system can deliver all kinds of small molecule RNAs and has strong versatility. Moreover, the preparation of viral vectors is much cheaper than the preparation of exosomes, proteins, polypeptides and the like, with good economy. The viral vector-based RNA delivery system provided in this example can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

### Example 2

On the basis of Example 1, a medicament is provided in this example. The medicament comprises a viral vector carrying an RNA fragment to be delivered, wherein the viral vector is capable of enriching and endogenously spontaneously forming a complex comprising the RNA fragment in a host organ or tissue, and the complex is capable of entering and binding to a target tissue, and delivering the RNA fragment into the target tissue. After the RNA fragment is delivered to the target tissue, it can inhibit the expression of its matching gene, thereby inhibiting the disease development in the target tissue.

Optionally, the RNA fragment comprises one, two or more RNA sequences with medical significance, and the RNA sequence is siRNA, shRNA or miRNA with medical significance.

In Figures 18-19, the two adenoviruses are represented as ADV1 and ADV2 respectively, and the six RNAs include siR^{E} (EGFR as the target gene), siR^{T} (TNC as the target gene), shR^{E} (EGFR as the target gene), siR^{T} (TNC as the target gene), miR-7 (EGFR as the target gene), and miR-133b (EGFR as the target gene). Figure 18 shows the expression levels of the corresponding proteins after intravenous injection of the 12 gene circuits constructed with the two adenoviral vectors. Figure 19 shows the protein levels after intravenous injection of delivery systems constructed with two adenoviral vectors and the RNA fragment composed of any two or three of six RNA sequences

The RNA sequences are specifically shown in Table 5 below.

**Table 5**

| **Name** | **Sequence** |
|---|---|
| siRE precursor sequence | ATACCTATTCCGTTACACACTGTTTTGGCCACTGACTGACAGT GTGTAGGAATAGGTAT |
| siRT precursor sequence | CACACAAGCCATCTACACATGGTTTTGGCCACTGACTGACCA TGTGTATGGCTTGTGTG |
| shRE precursor sequence | ATACCTATTCCGTTACACACTGTTTTGGCCACTGACTGACAGT GTGTAACGGAATAGGTAT |
| shRT precursor sequence | CACACAAGCCATCTACACATGGTTTTGGCCACTGACTGACCA TGTGTAGATGGCTTGTGTG |
| miR-7 | hsa-miR-7 |
| miR-133b | hsa-miR-133b |

Optionally, the viral vector comprises a promoter and a targeting tag, wherein the targeting tag is capable of forming a targeting structure of the complex in the host organ or tissue, the targeting tag is located on the surface of the complex, and the complex seeks for and binds to the target tissue through the targeting structure, and delivers the RNA fragment into the target tissue.

For explanations of the viral vector, RNA fragment, targeting tag, etc. in this example, please refer to Example 1 and will not be repeated here.

The medicament can enter the human body by oral administration, inhalation, subcutaneous injection, intramuscular injection or intravenous injection, and then be delivered to the target tissue through the viral vector-based RNA delivery system described in Example 1 to exert a therapeutic effect.

The medicament can be used to treat cancer, pulmonary fibrosis, colitis, obesity, cardiovascular disease caused by obesity, type 2 diabetes, Huntington's disease, Parkinson's disease, myasthenia gravis, Alzheimer's disease, or graft-versus-host disease.

The medicament in this example may also comprise a pharmaceutically acceptable carrier, which includes but is not limited to diluents, buffers, emulsions, encapsulating agents, excipients, fillers, adhesives, sprays, transdermal absorption agents, wetting agents, disintegrants, absorption accelerators, surfactants, colorants, flavoring agents, adjuvants, desiccants, adsorption carriers, etc.

The dosage form of the medicament provided in this example may be tablets, capsules, powders, granules, pills, suppositories, ointments, solutions, suspensions, lotions, gels, pastes, etc.

The medicament provided in this example uses a virus as a carrier, and as a mature injection, the viral vector has safety and reliability that have been fully verified, and has a very good druggability. The RNA sequence that exerts the final effect is encapsulated and transported by endogenous exosomes, with no immune responses, and there is no need to verify the safety of the exosomes. The delivery system can deliver all kinds of small molecule RNAs and has strong versatility. Moreover, the preparation of viruses is much cheaper than the preparation of exosomes, proteins, polypeptides and the like, with good economy. The medicament provided in the present application can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

### Example 3

On the basis of Example 1 or 2, use of a viral vector-based RNA delivery system in the manufacture of a medicament for treating a disease is provided in this example. The medicament is used for treating colitis. This example will specifically illustrate the effect of the viral vector-based RNA delivery system in the treatment of colitis through the following two experiments.

In the first experiment, we set up 3 experimental groups and 2 control groups. The experimental groups were AAV-CMV-siR^{TNF-α} (low) group, AAV-CMV-siR^{TNF-α} (medium) group, and AAV-CMV-siR^{TNF-α} (high) group; and the control groups were Normal group and AAV-CMV-scrR group.

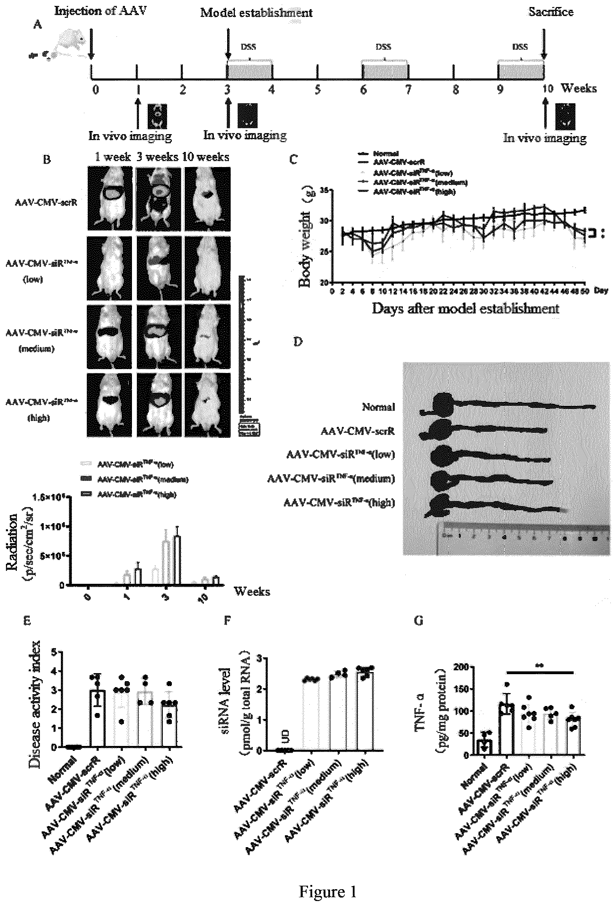

The experimental protocol is shown in Figure 1A. The AAV-CMV-siR^{TNF-α} (low) group, AAV-CMV-siR^{TNF-α} (medium) group, and AAV-CMV-siR^{TNF-α} (high) group used AAV-5 type adeno-associated virus with high affinity for the liver to encapsulate the TNF-α siRNA gene circuit (AAV-CMV-siR^{TNF-α}). Mice were injected with 25 µL, 50 µL and 100 µL of AAV solution with a titer of 10¹² V g/ml through the tail vein.

The in vivo expression of the AAV system was monitored by small animal in vivo imaging. The results are shown in Figure 1B. It can be seen that after 3 weeks, the AAV system was stably expressed in the body, especially in the liver. Among them, the AAV-CMV-siR^{TNF-α} (high) group had an average radiation up to 8.42* 10⁵ (p/sec/cm²/sr), and the expression site was in the liver, showing that the expression of the AAV system has a dose-dependent effect.

DSS-induced chronic colitis model was then constructed, during which weights was recorded every two days. The results are shown in Figure 1C. It can be seen that the AAV-encapsulated CMV-siR^{TNF-α} system can reduce the weight loss of mice with chronic colitis, and the mice in the three experimental groups had a significantly faster weight recovery than the AAV-CMV-scrR group during the inflammation remission period.

At the end of the tenth week of model construction, the in vivo expression of the AAV system was monitored by small animal in vivo imaging, and then the mice were sacrificed for observation of the colon. The results are shown in Figure 1D. It can be seen that the redness and swelling of the colon of mice in the AAV-CMV-siR ^{TNF-α} (low) group, AAV-CMV-siR^{TNF-α} (medium) group, and AAV-CMV-siR^{TNF-α} (high) group were alleviated to varying degrees, and the shortening of colon length caused by chronic inflammation also improved to varying degrees, wherein the improvement of inflammation in the AAV-CMV-siR^{TNF-α} (high) group was the most significant.

The disease index of mice in each group was scored and counted, and the results are shown in Figure 1E. It can be seen that the disease index of mice in the AAV-CMV-siR^{TNF-α}(high) group was smaller than that of AAV-CMV-siR^{TNF-α} (low) group, AAV-CMV-siR^{TNF-α} (medium) group and AAV-CMV-scrR group.

The levels of TNF-α siRNA in mice of each group were detected, and the results are shown in Figure 1F. It can be seen that the levels of TNF-α siRNA in mice of the three experimental groups were all high, while mice in the control group AAV-CMV-scrR group had almost no expression of TNF-α siRNA, showing that the above-mentioned AAV system can produce a certain amount of TNF-α siRNA.

The levels of TNF-α mRNA in mice of each group were detected, and the results are shown in Figure 1G. It can be seen that the levels of TNF-α mRNA in mice of the Normal group and the three experimental groups were all low, while the level of TNF-α mRNA in mice of the AAV-CMV-scrR group was high, showing that the AAV system can reduce the expression and secretion of TNF-α in the colon.

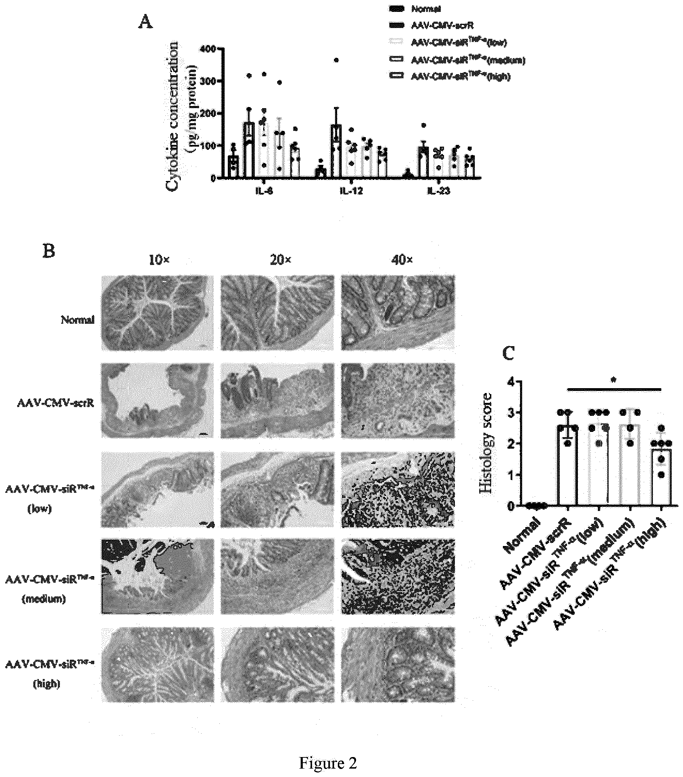

The pro-inflammatory cytokines IL-6, IL-12 and IL-23 in the mouse colon were detected, and the results are shown in Figure 2A. It can be seen that mice in the Normal group and the AAV-CMV-siR^{TNF-α} (high) group secreted the least pro-inflammatory cytokines, and mice in the AAV-CMV-scrR group secreted the most pro-inflammatory cytokines.

HE staining and pathological scoring statistics were performed on mouse colon sections, and the results are shown in Figure 2B and Figure 2C. It can be seen that mice in the experimental groups, especially mice in the AAV-CMV-siR^{TNF-α} (high) group, had higher colon mucosal integrity, shallower immune cell infiltration, and significantly less colonic crypt abscesses and colon congestion and bleeding compared to the AAV-CMV-scrR group.

The above experiments show that the use of AAV with high affinity for the liver to encapsulate the CMV-siR^{TNF-α} circuit can achieve long-term expression of TNF-α siRNA and long-term TNF-α silencing, and can alleviate colitis to a certain extent, with great drug potential and clinical research value.

In the second experiment, we set up three experimental groups and two control groups. Wherein, the experimental groups were AAV-CMV-siR^{T+B+I} (low) group, AAV-CMV-siR^{T+B+I} (medium) group, and AAV-CMV-siR^{T+B+I} (high) group; and the control groups were Normal group and AAV-CMV-scrR group.

The AAV-CMV-siR^{T+B+I} (low) group, AAV-CMV-siR^{T+B+I} (medium) group, and AAV-CMV-siR^{T+B+I} (high) group used AAV-5 type adeno-associated virus with high affinity for the liver to encapsulate the TNF-α siRNA, B7-siRNA and Integrin α4 siRNA element tandem drug delivery system (AAV-CMV-siR^{T+B+I}). Mice were injected with 25 µL, 50 µL and 100 µL of AAV solution with a titer of 10¹² V g/ml through the tail vein.

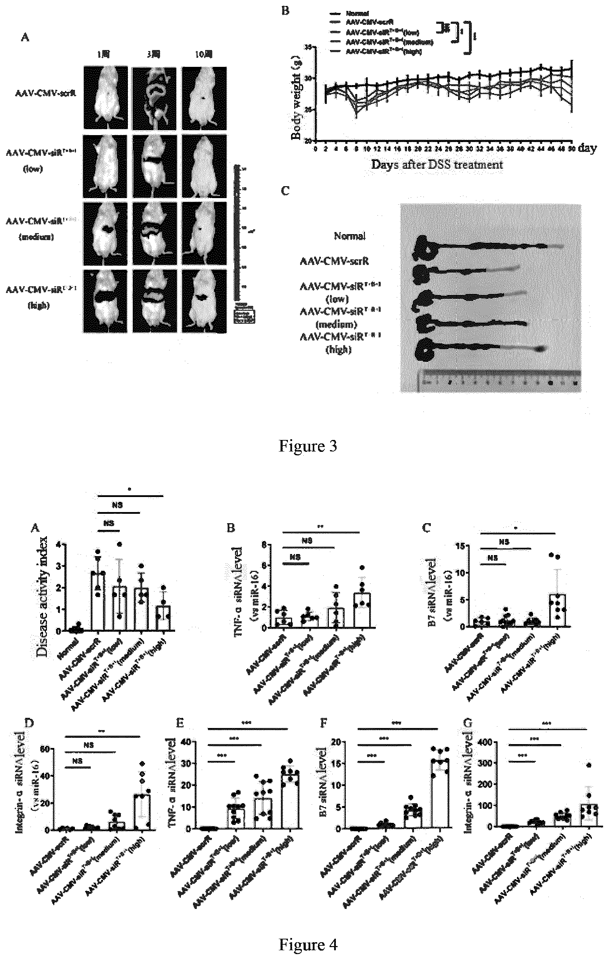

The in vivo expression of the AAV system was monitored by small animal in vivo imaging. The results are shown in Figure 3A. It can be seen that after 3 weeks, the AAV system was stably expressed in the body, especially in the liver, and that the expression of the AAV system has a dose-dependent effect.

DSS-induced chronic colitis model was then constructed, during which weights was recorded every two days. The results are shown in Figure 3B. It can be seen that the AAV-encapsulated CMV-siR^{T+B+I} system can reduce the weight loss of mice with chronic colitis, and the mice in the three experimental groups had a significantly faster weight recovery than the AAV-CMV-scrR group during the inflammation remission period.

At the end of the tenth week of model construction, the in vivo expression of the AAV system was monitored by small animal in vivo imaging, and then the mice were sacrificed for observation of the colon. The results are shown in Figure 3C. It can be seen that the redness and swelling of the colon of mice in the AAV-CMV-siR^{T+B+I} (low) group, AAV-CMV-siR^{T+B+I} (medium) group, and AAV-CMV-siR^{T+B+I} (high) group were alleviated to varying degrees, and the shortening of colon length caused by chronic inflammation also improved to varying degrees, wherein the improvement of inflammation in the AAV-CMV-siR^{T+B+I} (high) group was the most significant.

The disease index of mice in each group was scored and counted, and the results are shown in Figure 4A. It can be seen that the disease index of mice in the AAV-CMV-siR^{T+B+I} (high) group was smaller than that of AAV-CMV-siR^{T+B+I} (low) group, AAV-CMV-siR^{T+B+I} (medium) group and AAV-CMV-scrR group.

The levels of TNF-α siRNA, B7 siRNA and integrin α4 siRNA were detected in mouse plasma, and the results are shown in Figure 4B, Figure 4C, and Figure 4D. It can be seen that AAV-encapsulated CMV-siR^{T+B+I} system produced a certain amount of siRNA in mouse plasma with a dose-dependent effect.

The levels of TNF-α siRNA, B7 siRNA and integrin α4 siRNA were detected in mouse liver, and the results are shown in Figure 4E, Figure 4F, and Figure 4G. It can be seen that the AAV-encapsulated CMV-siR^{T+B+I} system produced a certain amount of siRNA in mouse liver with a dose-dependent effect.

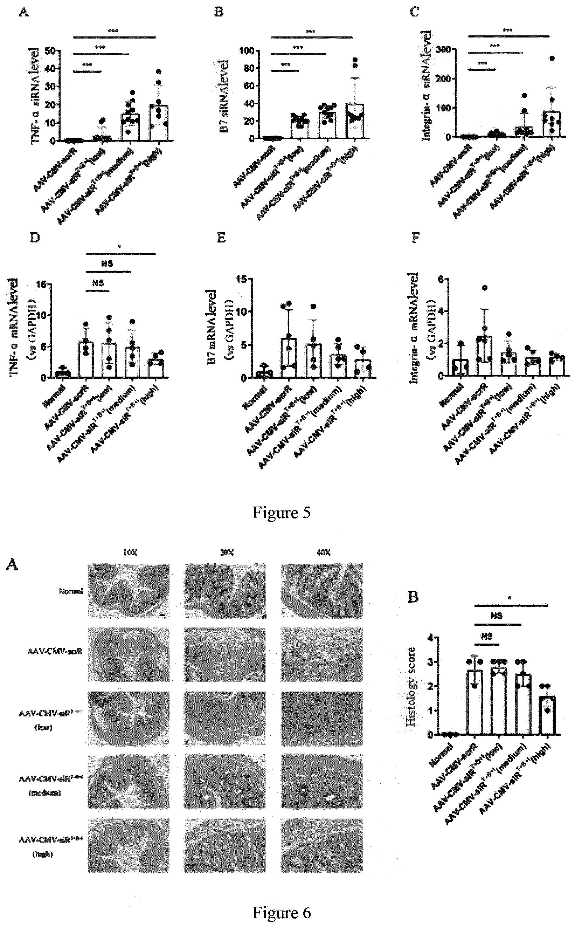

The levels of TNF-α siRNA, B7 siRNA and integrin α4 siRNA were detected in mouse colon, and the results are shown in Figure 5A, Figure 5B, and Figure 5C. It can be seen that the AAV-encapsulated CMV-siR^{T+B+I} system produced a certain amount of siRNA in mouse colon with a dose-dependent effect.

The levels of TNF-α mRNA, B7 mRNA and integrin α4 mRNA were detected in mouse colon, and the results are shown in Figure 5D, Figure 5E, and Figure 5F. It can be seen that the AAV-encapsulated CMV-siR^{T+B+I} system significantly reduced expression of TNF-α mRNA, B7 mRNA and integrin α4 mRNA.

HE staining and pathological scoring statistics were performed on mouse colon sections, and the results are shown in Figure 6A and Figure 6B. It can be seen that mice in the experimental groups, especially mice in the AAV-CMVsiR^{T+B+I} (high) group, had higher colon mucosal integrity, shallower immune cell infiltration, and significantly less colonic crypt abscesses and colon congestion and bleeding compared to the AAV-CMV-scrR group.

The above experiments show that the use of AAV with high affinity for the liver to encapsulate the CMV-siR^{T+B+I} circuit can achieve long-term expression of TNF-α siRNA, B7 siRNA and integrinα4 siRNA and multiple target gene silencing, and can significantly alleviate the degree of colon inflammation, with great drug potential and clinical research value.

### Example 4

On the basis of Example 1 or 2, use of a viral vector-based RNA delivery system in the manufacture of a medicament for treating a disease is provided in this example. The medicament is used for treating lung cancer. This example will specifically illustrate the effect of the viral vector-based RNA delivery system in the treatment of lung cancer through the following four experiments.

In the first experiment, we used AAV-5 adeno-associated virus with high affinity for the liver to encapsulate the EGFR siRNA system (AAV-CMV-EGFR siRNA) and the KRAS siRNA system (AAV-CMV-KRAS siRNA). Mice were injected with 100 µL of AAV solution with a titer of 10¹² V g/ml through the tail vein. The in vivo expression of the AAV system was monitored by small animal in vivo imaging. After 3 weeks, it was found that the AAV system was stably expressed in the body, especially in the liver.

In the second experiment, one experimental group and two control groups were set up, wherein the experimental group was the AAV-CMVKRAS-siRNA group, and the control groups were the PBS group and the AAV-CMV-scrR group.

The same number of mice were selected from each group. Mouse lung cancer cells (LLC cells) were injected into the mice, and CT scanning technology was used to observe the progress of mouse model establishment. After 30 days, the successfully established mice were administered once every two days. Specifically, mice in the PBS group/AAV CMV scrR group/AAV-CMV-KRAS siRNA group were injected with PBS buffer/AAV-CMV-scrR/AAV-CMV-KRAS siRNA once every two days for treatment. Survival analysis and tumor evaluation were performed on the mice. Treatment was stopped after 7 administrations.

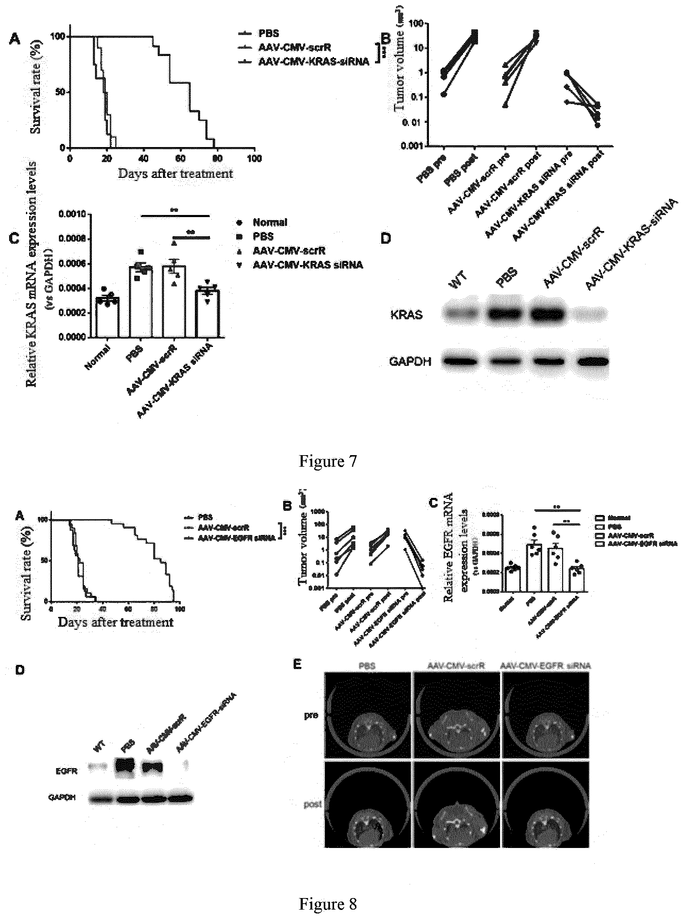

The survival of mice in each group was counted within 100 days after treatment, and the results are shown in Figure 7A. It can be seen that the survival rates of mice in the PBS group and the AAV-CMV-scrR group were almost the same, while mice in the AAV-CMV-KRAS siRNA group had the highest survival rate.

CT scanning was performed on mice in each group before and after administration. 3D modeling of mouse lung tissue was performed based on the CT images, and the tumor volume was calculated. The results are shown in Figure 7B. In Figure 7B, "PBS pre" indicates the PBS group before administration, "PBS post" indicates the PBS group after administration; "AAV-CMV-scrR pre" indicates the AAV-CMV-scrR group before administration; "AAV-CMV-scrR post" indicates the AAV-CMV-scrR group after administration; "AAV-CMV-KRAS-siRNA pre" indicates the AAV-CMV-KRAS siRNA group before administration; and "AAV-CMV-KRAS-siRNA post" indicates the AAV-CMV-KRAS siRNA group after administration. It can be seen that the tumor volume of mice in the AAV-CMV-KRAS siRNA group decreased significantly after administration, while the tumor volume of mice in the PBS group and AAV-CMV-scrR group not only failed to decrease after administration, but also increased to varying degrees.

The expression levels of KRAS protein and mRNA in the lung of mice in each group were detected by RT-qPCR and Western blotting, respectively, and the results are shown in Figure 7C and Figure 7D. The results showed that the expression of KRAS protein and mRNA in the lung of mice in the AAV-CMV-KRAS siRNA group was reduced compared with the control groups.

The above experiments show that AAV-CMV-KRAS siRNA had a significant therapeutic effect on lung cancer tumors in mice.

In the third experiment, one experimental group and two control groups were set up, wherein the experimental group was the AAV-CMVEGFR-siRNA group, and the control groups were the PBS group and the AAV-CMV-scrR group.

EGFR-DEL19 mouse model was constructed and fed with doxycycline feed to induce tumor occurrence. After 30 days, the successfully constructed mice were administered once every two days. Specifically, mice in the PBS group/AAV CMV scrR group/AAV CMV EGFR siRNA group were injected with PBS buffer/AAV CMV scrR/AAV CMV EGFR siRNA once every two days for treatment. Survival analysis and tumor evaluation were performed on the mice. Treatment was stopped after 7 administrations.

The survival of mice in each group was counted within 100 days after treatment, and the results are shown in Figure 8A. It can be seen that the survival rates of mice in the PBS group and the AAV-CMV-scrR group were almost the same, while mice in the AAV-CMV-EGFR siRNA group had the highest survival rate.

CT scanning was performed on mice in each group before and after administration, and the CT images are shown in Figure 8E. 3D modeling of mouse lung tissue was performed based on the CT images, and the tumor volume was calculated. The results are shown in Figure 8B. In Figure 8B, "PBS pre" indicates the PBS group before administration, "PBS post" indicates the PBS group after administration; "AAV-CMV-scrR pre" indicates the AAV-CMV-scrR group before administration; "AAV-CMV-scrR post" indicates the AAV-CMV-scrR group after administration; "AAV-CMV-EGFR-siRNA pre" indicates the AAV-CMV-EGFR siRNA group before administration; and "AAV-CMV-EGFR-siRNA post" indicates the AAV-CMV-EGFR siRNA group after administration. It can be seen that the tumor volume of mice in the AAV-CMV-EGFR siRNA group decreased significantly after administration, while the tumor volume of mice in the PBS group and AAV-CMV-scrR group not only failed to decrease after administration, but also increased to varying degrees.

The expression levels of EGFR protein and mRNA in the lung of mice in each group were detected by RT-qPCR and Western blotting, respectively, and the results are shown in Figure 8C and Figure 8D. The results showed that the expression of EGFR protein and mRNA in the lung of mice in the AAV-CMV-EGFR siRNA group was reduced compared with the control groups.

The above experiments show that AAV-CMV-EGFR siRNA had a significant therapeutic effect on EGFR-mutated lung cancer tumors in mice.

In the fourth experiment, two experimental groups and two control groups were set up, wherein the experimental groups were the AAV-CMV-KRAS-siRNA group the AAV-CMV-EGFR-siRNA group, and the control groups were the PBS group and the AAV-CMV-scrR group.

EGFR-DEL19 mouse model was constructed and fed with doxycycline feed to induce tumor occurrence. After 30 days, the successfully constructed mice were administered once every two days. Specifically, mice in the PBS group/AAV-CMV-scrR group/AAV-CMV-EGFR siRNA group/AAV-CMV-KRAS siRNA group were injected with PBS buffer/AAV-CMV-scrR/AAV-CMV-EGFR siRNA/AAV-CMV-KRAS siRNA once every two days for treatment.

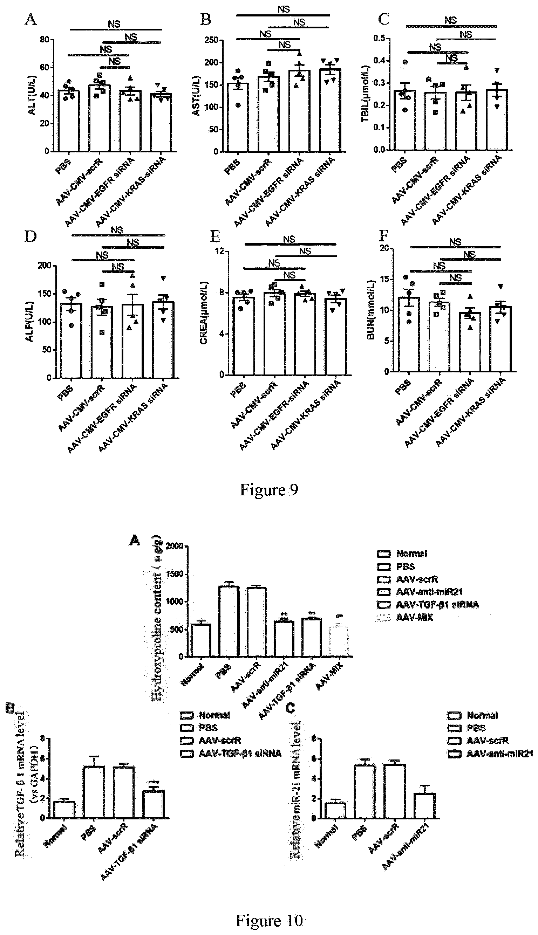

After treatment, alanine aminotransferase (ALT), aspartate aminotransferase (AST), total bilirubin (TBIL), serum alkaline phosphatase (ALP), creatinine (CREA) and blood urea nitrogen (BUN) were detected in each group of mice, and the results are shown in Figure 9A-Figure 9F. It can be seen that the contents of the above enzymes in the PBS group, AAV-CMV-scrR group, AAV-CMV-EGFR siRNA group and AAV-CMV-KRAS siRNA group were almost the same.

The above experiments demonstrate that the EGFR siRNA system (AAV-CMV-EGFR siRNA) and KRAS siRNA system (AAV-CMV-KRAS siRNA) encapsulated with AAV-5 type adeno-associated virus with high affinity for the liver is safe and reliable and will not produce negative effects, which is suitable for large-scale promotion and application.

### Example 5

On the basis of Example 1 or 2, use of a viral vector-based RNA delivery system in the manufacture of a medicament for treating a disease is provided in this example. The medicament is used for treating pulmonary fibrosis. This example will specifically illustrate the effect of the viral vector-based RNA delivery system in the treatment of pulmonary fibrosis through the following experiments.

In this example, AAV-5 type adeno-associated virus with high affinity for the liver was used to encapsulate anti-miR-21/TGF-β1 siRNA/anti-miR-21+TGF-β1 siRNA system to obtain AAV-anti-miR21/AAV-TGF-β1 siRNA/AAV-MIX, respectively. Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice were then selected for modeling. After the modeling was successful, PBS buffer/AAV-scrR/AAV-anti-miR21/AAV-TGF-β1 siRNA/AAV-MIX (10 mg/kg) was injected into mice to obtain PBS group/AAV-scrR group/AAV-anti-miR21 group/AAV-TGF-β1 siRNA group/AAV-MIX group, respectively.

The relative TGF-β1 mRNA level was detected in mice in the Normal group, PBS group, AAV-scrR group, and AAV-TGF-β1 siRNA group, and the results are shown in Figure 10B. It can be seen that the relative TGF-β1 mRNA level of mice in the AAV-TGF-β1 siRNA group was relatively low.

The relative miR21 mRNA level was detected in mice in the Normal group, PBS group, AAV-scrR group, and AAV-anti-miR21 group, and the results are shown in Figure 10C. It can be seen that the relative miR21 mRNA level of mice in the AAV-anti-miR21 group was relatively low.

Hydroxyproline is the main component of collagen, and its content reflects the degree of pulmonary fibrosis. The hydroxyproline content of mice in each group was detected, and the results are shown in Figure 10A. It can be seen that mice in the PBS group and the AAV-scrR group had the highest hydroxyproline content, while mice in the AAV-anti-miR21 group, AAV-TGF-β1 siRNA group, and AAV-MIX group all had low hydroxyproline content, indicating that pulmonary fibrosis was inhibited in mice in the AAV-anti-miR21 group, AAV-TGF-β1 siRNA group, and AAV-MIX group.

### Example 6

On the basis of Example 1 or 2, use of a viral vector-based RNA delivery system in the manufacture of a medicament for treating a disease is provided in this example. The medicament is used for treating glioblastoma. This example will specifically illustrate the effect of the viral vector-based RNA delivery system in the treatment of glioblastoma through the following experiments.

In this example, AAV-5 type adeno-associated virus with high affinity for the liver was used to encapsulate the EGFR siRNA system (AAV-CMV-RVG-siR^{E}) and the EGFR siRNA and TNC siRNA system (AAV-CMV-RVG-siR^{E+T}). Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice were selected and injected with glioblastoma cells (U-87 MG-Luc cells). From the 7th day to the 21st day, mice were injected with PBS buffer/AAV-CMV-scrR/AAV-CMV-RVG-siR^{E}/AAV-CMV-RVG-siR^{E+T} (5 mg/kg) every two days for treatment, to obtain PBS group/AAV-scrR group/AAV-CMV-RVG-siR^{E} group/AAV-CMV-RVG-siR^{E+T} group, respectively.

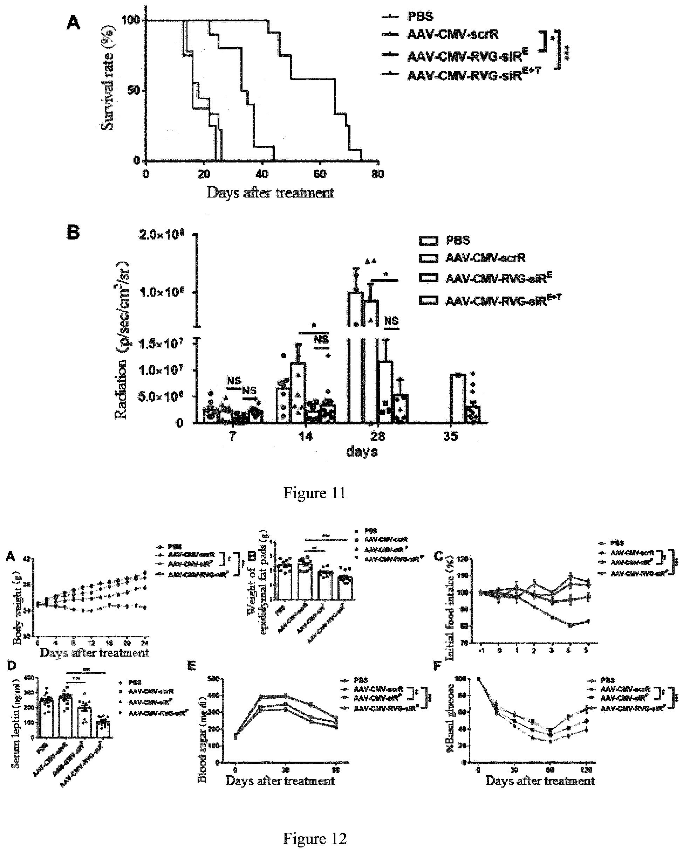

Survival analysis was performed on mice in each group, and the survival rate of mice in each group was counted at 20 days, 40 days, 60 days, and 80 days after receiving treatment. The results are shown in Figure 11A. It can be seen that mice in the AAV-CMV-RVG-siR^{E+T} group had the longest survival time, followed by the AAV-CMV-RVG-siR^{E} group.

Tumor evaluation was performed on mice in each group. Specifically, BLI in vivo imaging was performed on mice on days 7, 14, 28, and 35. The results are shown in Figure 11B. It can be seen that the AAV-CMV-RVG-siR^{E+T} group had the most significant inhibitory effect on glioblastoma in mice.

### Example 7

On the basis of Example 1 or 2, use of a viral vector-based RNA delivery system in the manufacture of a medicament for treating a disease is provided in this example. The medicament is used for treating obesity. This example will specifically illustrate the effect of the viral vector-based RNA delivery system in the treatment of obesity through the following experiments.

AAV-5 type adeno-associated virus with high affinity for the liver was used to encapsulate the PTP1B siRNA system (AAV-CMV-siR^{P}/AAV-CMV-RVG-siR^{P}). Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

C57BL/6 mice were selected and injected with PBS buffer/AAV-CMV-scrR/AAV-CMV-siR^{P}/AAV-CMV-RVG-siR^{P} 12 weeks later, and once every two days for 24 days to obtain PBS group/AAV-CMV-scrR group/AAV-CMV-siR^{P} group/AAV-CMV-RVG-siR^{P} group, respectively. The changes in body weight, weight of epididymal fat pads, initial food intake, serum leptin content, blood sugar content, basal glucose content, serum total cholesterol (TC), triglyceride (TG), low-density lipoprotein protein (LDL), body length, and food intake of mice in each group were detected and counted. The results are as follows.

As shown in Figure 12A, the figure shows the comparison of the body weights of mice in each group. It can be seen that the body weight of mice in the AAV-CMV-RVG-siR^{P} group was the most stable.

As shown in Figure 12B, the figure shows the comparison of the weights of the epididymal fat pads of mice in each group. It can be seen that the weight of the epididymal fat pads of mice in the AAV-CMV-RVG-siR^{P} group was the lightest.

As shown in Figure 12C, the figure shows the comparison of the initial food intake curves of mice in each group. It can be seen that mice in the AAV-CMV-RVG-siR^{P} group had the least food intake.

As shown in Figure 12D, this figure shows the comparison of serum leptin levels in mice of each group. It can be seen that the serum leptin levels of mice in the AAV-CMV-RVG-siR^{P} group was the lowest.

As shown in Figure 12E, this figure shows the comparison of blood glucose change curves of mice in each group. It can be seen that the blood glucose level of mice in the AAV-CMV-RVG-siR^{P} group was the lowest.

As shown in Figure 12F, this figure shows the comparison of the basal glucose change curves of mice in each group. It can be seen that mice in the AAV-CMV-RVG-siR^{P} group had the lowest basal glucose content.

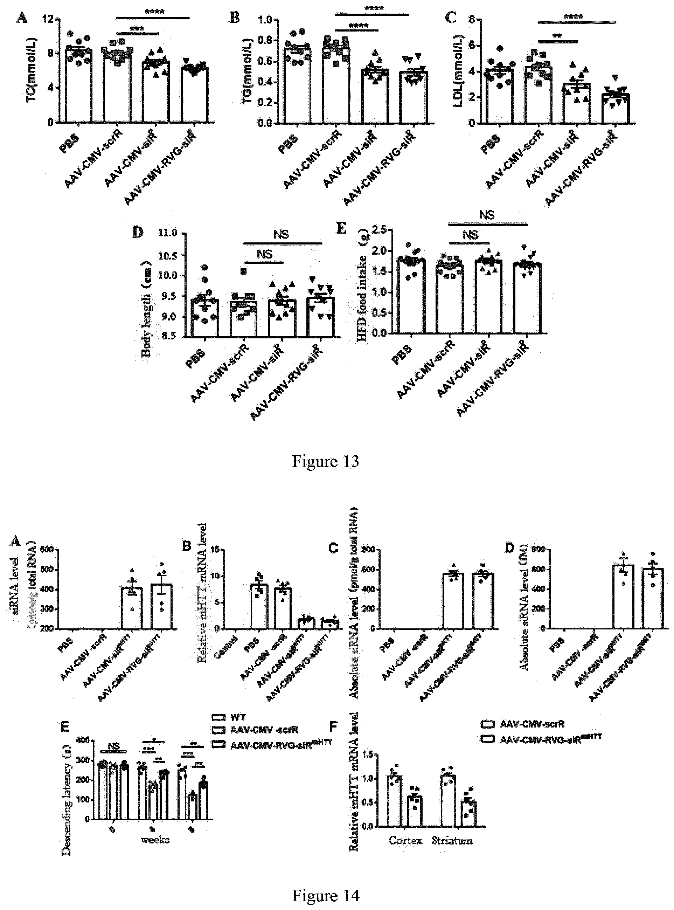

As shown in Figure 13A-Figure 13C, the three figures show the comparison of serum total cholesterol (TC), triglyceride (TG), and low-density lipoprotein (LDL) of mice in each group. It can be seen that mice in the AAV-CMV-RVG-siR^{P} group had the lowest TC, TG, and LDL.

As shown in Figure 13D, the figure shows the comparison of the body lengths of the mice in each group, it can be seen that the body lengths of the mice in the four groups were almost the same.

As shown in Figure 13E, the figure shows the comparison of the HFD food intake of the mice in each group, it can be seen that the HFD food intake of mice in the four groups was almost the same.

The above experiments can demonstrate that AAV-CMV-siR^{P} and AAV-CMV-RVG-siR^{P} had a certain degree of inhibitory effect on obesity.

### Example 8

On the basis of Example 1 or 2, use of a viral vector-based RNA delivery system in the manufacture of a medicament for treating a disease is provided in this example. The medicament is used for treating Huntington's disease. This example will specifically illustrate the effect of the viral vector-based RNA delivery system in the treatment of Huntington's disease through the following experiments.

In this example, AAV-5 type adeno-associated virus with high affinity for the liver was used to encapsulate the HTT siRNA system (AAV-CMV-RVG-siR^{mHTT}). Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice was then selected for modeling. After the completion of modeling, mice were injected with PBS buffer/AAV-CMV-scrR/AAV-CMV-siR^{mHTT}/AAV-CMV-RVG-siR^{mHTT} to obtain PBS group/AAV-CMV-scrR group/AAV-CMV-siR^{mHTT} group/AAV-CMV-RVG-siR^{mHTT} group. After the above solution was injected into the tail vein, the plasma exosomes were isolated, labeled with PKH26 dye, and co-cultured with cells to observe the absorption of exosomes by cells. The results are as follows.

As shown in Figure 14A, the figure shows the comparison of siRNA levels in plasma exosomes of mice in each group. It can be seen that the levels of siRNA in plasma exosomes of mice in the AAV-CMV-siR^{mHTT} group and AAV-CMV-RVG-siR^{mHTT} group were higher.

As shown in Figure 14B, this figure shows the comparison of the relative mHTT mRNA levels of mice in each group after co-culturing the mouse plasma exosomes with cells. It can be seen that the mice in the AAV-CMV-siR^{mHTT} group and AAV-CMV-RVG-siR^{mHTT} group had lower relative mHTT mRNA levels, indicating that AAV-CMV-siR^{mHTT} and AAV-CMV-RVG-siR^{mHTT} could reduce HTT mRNA levels, meaning siRNA assembled into exosomes could still exert gene silencing function.

As shown in Figure 14C, this figure shows the comparison of absolute levels of siRNA in mouse liver. It can be seen that the absolute levels of siRNA of mice in the AAV-CMV-siR^{mHTT} group and AAV-CMVRVG-siR^{mHTT} group were higher.

As shown in Figure 14D, this figure shows the comparison of absolute levels of siRNA in mouse plasma. It can be seen that the absolute levels of siRNA of mice in the AAV-CMV-siR^{mHTT} group and AAV-CMVRVG-siR^{mHTT} group were higher.

As shown in Figure 14E, this figure shows the comparison of the descending latency of wild-type mice (WT) and mice in the AAV-CMV-scrR group and AAV-CMV-RVG-siR^{mHTT} group. It can be seen that at 0 weeks, the descending latency of mice in the three groups was relatively consistent. At the 4th and 8th weeks, the descending latency of mice in the CMV-scrR group was the shortest.

As shown in Figure 14F, this figure shows the comparison of the relative mHTT mRNA levels in the cortex and striatum of mice in the AAV-CMV-scrR group and AAV-CMV-RVG-siR^{mHTT} group. It can be seen that whether in the cortex or the striatum, the mHTT mRNA levels of mice in the AAV-CMV-RVG-siR^{mHTT} group were lower than those in the AAV-CMV-scrR group.

The above experiments can demonstrate that intravenous injection of AAV-CMV-RVG-siR^{mHTT} can help reduce mHTT protein and toxic aggregates in the striatum and cortex, thereby exerting a therapeutic effect on Huntington's disease.

The terms "upper", "lower", "front", "back", "left", "right", etc. used herein are only used to express the relative positional relationship between related parts, but not to limit the absolute position of these related parts.

The terms "first", "second", etc. used herein are only used to distinguish each other, but do not indicate the degree of importance, order, or the prerequisite for each other's existence.

The terms "equal", "identical", etc. used herein are not limitations in a strict mathematical and/or geometric sense, but also include errors that are understandable by those skilled in the art and allowed in manufacturing or use.

Unless otherwise stated, numerical ranges herein include not only the entire range within both endpoints, but also several subranges subsumed therein.

The preferred specific embodiments and examples of the present application have been described in detail above in conjunction with the accompanying drawings. However, the present application is not limited to the above-mentioned embodiments and examples. Within the scope of knowledge possessed by those skilled in the art, various changes can be made without departing from the concept of the present application.

## Claims

1. A viral vector-based RNA delivery system, comprising a viral vector carrying an RNA fragment to be delivered, wherein the viral vector is capable of enriching and endogenously spontaneously forming a complex comprising the RNA fragment in a host organ or tissue, and the complex is capable of entering and binding to a target tissue, and delivering the RNA fragment into the target tissue.

2. The viral vector-based RNA delivery system according to claim 1, wherein the viral vector is an adenovirus-associated virus.

3. The viral vector-based RNA delivery system according to claim 2, wherein the viral vector is adenovirus-associated virus type 5, adenovirus-associated virus type 8 or adenovirus-associated virus type 9.

4. The viral vector-based RNA delivery system according to claim 1, wherein the RNA fragment comprises one, two or more RNA sequences with medical significance, and the RNA sequence is siRNA, shRNA or miRNA with medical significance.

5. The viral vector-based RNA delivery system according to claim 1, wherein the viral vector comprises a promoter and a targeting tag, wherein the targeting tag is capable of forming a targeting structure of the complex in the host organ or tissue, the targeting tag is located on the surface of the complex, and the complex seeks for and binds to the target tissue through the targeting structure, and delivers the RNA fragment into the target tissue.

6. The viral vector-based RNA delivery system according to claim 5, wherein the viral vector comprises a circuit selected from the group consisting of promoter-RNA fragment, promoter-targeting tag, and promoter-RNA fragment-targeting tag, or a combination thereof; and the viral vector comprises at least an RNA fragment and a targeting tag, wherein the RNA fragment and the targeting tag are located in the same circuit or in different circuits.

7. The viral vector-based RNA delivery system according to claim 6, wherein the viral vector further comprises a flanking sequence, a compensation sequence and a loop sequence that facilitate the correct folding and expression of the circuit, and the flanking sequence comprises 5' flanking sequence and 3' flanking sequence; and
the viral vector comprises a circuit selected from the group consisting of 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence, or a combination thereof.

8. The viral vector-based RNA delivery system according to claim 7, wherein the 5' flanking sequence has a sequence that is identical to or more than 80% homologous with ggatcctggaggcttgctgaaggctgtatgctgaattc;
the loop sequence has a sequence that is identical to or more than 80% homologous with gttttggccactgactgac;
the 3' flanking sequence has a sequence that is identical to or more than 80% homologous with accggtcaggacacaaggcctgttactagcactcacatggaacaaatggcccagatctggccgcactcgag; and
the compensation sequence has a reverse complementary sequence to the RNA fragment in which any 1 to 5 bases have been deleted.

9. The viral vector-based RNA delivery system according to claim 6, wherein in the case where the viral vector comprises two or more of the circuits, adjacent circuits are linked via a sequence composed of sequences 1-3;
wherein, sequence 1 is CAGATC, sequence 2 is a sequence of 5-80 bases, and sequence 3 is TGGATC.

10. The viral vector-based RNA delivery system according to claim 9, wherein in the case where the viral vector comprises two or more of the gene circuits, adjacent gene circuits are linked via sequence 4 or a sequence with more than 80% homology to sequence 4;
wherein, sequence 4 is CAGATCTGGCCGCACTCGAGGTAGTGAGTCGACCAGTGGATC.

11. The viral vector-based RNA delivery system according to claim 1, wherein the organ or tissue is liver, and the complex is an exosome.

12. The viral vector-based RNA delivery system according to claim 5, wherein the targeting tag is a targeting peptide or targeting protein with targeting function.

13. The viral vector-based RNA delivery system according to claim 12, wherein the targeting peptide is selected from the group consisting of RVG targeting peptide, GE11 targeting peptide, PTP targeting peptide, TCP-1 targeting peptide, and MSP targeting peptide; and
the targeting protein is selected from the group consisting of RVG-LAMP2B fusion protein, GE11-LAMP2B fusion protein, PTP-LAMP2B fusion protein, TCP-1-LAMP2B fusion protein, and MSP-LAMP2B fusion protein.

14. The viral vector-based RNA delivery system according to claim 5, wherein the RNA sequence is 15-25 nucleotides in length.

15. The viral vector-based RNA delivery system according to claim 14, wherein the RNA sequence has a sequence that is identical to, more than 80% homologous with, or of a nucleic acid molecule encoding a sequence selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, siRNA of α-synuclein gene, and a combination thereof.

16. The viral vector-based RNA delivery system according to claim 1, wherein the delivery system is a delivery system for use in a mammal including human.

17. Application of the viral vector-based RNA delivery system according to any one of claims 1 to 16 in the manufacture of a medicament for treating a disease.

18. The application according to claim 17, wherein the medicament is administered by oral administration, inhalation, subcutaneous injection, intramuscular injection, or intravenous injection.

19. The application according to claim 17, wherein the disease is a cancer, pulmonary fibrosis, colitis, obesity, cardiovascular disease caused by obesity, type 2 diabetes, Huntington's disease, Parkinson's disease, myasthenia gravis, Alzheimer's disease, or graft-versus-host disease.
